# EUROPEAN PATENT SPECIFICATION

(11) **EP 1 438 392 B1**
(45) Date of publication and mention of the grant of the patent: **29.07.2015**
(21) Application number: 02778303.4
(22) Date of filing: 20.09.2002
(51) Int. Cl.: A61K 35/12, C12N 5/077, C12N 5/0775, C12N 5/0797

(54) **CELL POPULATIONS WHICH CO-EXPRESS CD49C AND CD90**
ZELLPOPULATIONEN, DIE CD49C UND CD90 EXPRIMIEREN
POPULATIONS CELLULAIRES QUI CO-EXPRIMENT CD49C ET CD90

(30) Priority: 21.09.2001 US 960244
(43) Date of publication of application: 21.07.2004
(73) Proprietor: Garnet BioTherapeutics, Inc, Malvern, PA 19335 (US)
(72) Inventor: HO, Tony, W., Berwyn, PA 19312 (US); KOPEN, Gene, C., Wynnewood, PA 19096 (US); RIGHTER, William, F., Ridley Park, PA 19078 (US); RUTKOWSKI, J., Lynn, Wynnewood, PA 19096 (US); HERRING, W., Joseph, Valley Forge, PA 19481 (US); RAGAGLIA, Vanessa, Malvern, PA 19355 (US); WAGNER, Joseph, West Chester, PA 19380 (US)
(74) Representative: Lock, Graham James
(86) International application number: PCT/US2002/029971
(87) International publication number: WO 2003/025149

(56) References cited:
- WO-A2-01/11011
- US-A- 5 837 539
- LODIE T A ET AL: "Systematic analysis of reportedly distinct populations of multipotent bone marrow-derived stem cells reveals a lack of distinction" TISSUE ENGINEERING, LARCHMONT, NY, US, vol. 8, no. 5, October 2002 (2002-10), pages 739-751, XP002306489 ISSN: 1076-3279
- PITTENGER ET AL.: 'Multilineage potential of adult human mesenchymal stem cells' SCIENCE vol. 284, 02 April 1999, pages 143 - 147, XP002942313
- COOPER ET AL.: 'Incipient analysis of mesenchymal stem-cell-derived osteogenesis' J. DENT. RES. vol. 80, no. 1, 2001, pages 314 - 320, XP008008738
- BOS ET AL.: 'P21cip1 rescues human mesenchymal stem cells from apoptosis induced by low-density culture' CELL TISSUE RES. vol. 293, 1998, pages 464 - 470, XP000926493
- GARTEL ET AL.: 'Transcriptional regulation of the p21(waf1/cip1) gene' EXPERIMENTAL CELL RESEARCH vol. 246, 1999, pages 280 - 289, XP002972657

## Description

### BACKGROUND OF THE INVENTION

A number of conditions and diseases of the central nervous system (i.e., brain and spinal cord), peripheral nervous system and heart adversely affect humans. These conditions and diseases include, for example, spinal cord injury, amyotrophic lateral sclerosis (ALS), Parkinson's disease, stroke, traumatic brain injury, brain tumors, Fabry Disease, congestive heart failure and myocardial infarction. Clinical management strategies, for example, frequently focus on the prevention of further damage or injury rather than replacement or repair of the damaged tissue (e.g., neurons, glial cells, cardiac muscle); include treatment with exogenous steroids and synthetic, non-cellular pharmaceutical drugs; and have varying degrees of success which may depend on the continued administration of the steroid or synthetic drug.

For example, the majority of spinal cord injuries are compression injuries with the remaining cases involving complete transection of the spinal cord. Current therapeutic treatments for spinal cord injury include the prevention of additional spinal cord injury by physically stabilizing the spine through surgical and non-surgical procedures and by inhibiting the inflammatory response with steroidal therapy. Thus, there is a need to develop new, improved and effective methods of treatment for diseases and conditions, in particular, neurological and cardiac diseases and conditions, in humans.

WO 01/04 268 discloses a cell population expressing CD49c and CD90.

### SUMMARY OF THE INVENTION

The present invention relates to cell populations which co-express CD49c and CD90 and methods of treating conditions, such as neurological or cardiac conditions, in humans with these populations of cells.

The present invention provides a method of making an isolated cell population derived from human bone marrow, wherein between 100% to 90% of the cells of the cell population co-express CD49c and CD90, and wherein the cell population has a doubling time of less than 30 hours, comprising the steps of: a) culturing a source of the cell population under a low oxygen condition of less than 15% oxygen to produce an adherent cell population; and, b) culturing the adherent cell population at a seeding density of less than 2500 cells/cm².

The present invention also provides a method as defined above, wherein prior to steps a) and b), the method further comprises the steps of: a) lysing the red blood cell component of a human bone marrow aspirate; b) seeding the non-lysed bone marrow cells in a tissue culturing device; c) allowing the non-lysed bone marrow cells to adhere to a surface of the tissue culturing device; and wherein steps a) and b) of the method as defined above correspond to steps d) and e) comprising: d) culturing the adherent cells under a 5% oxygen condition; and e) passaging the adherent cells at a seeding density of 30 cells/cm².

The present invention alternatively also provides a method as defined above, wherein prior to steps a) and b), the method further comprises the steps of: a) selecting a fractionated source of the cell population of a human bone marrow aspirate by passage through a density gradient; b) seeding the fractionated cells in a tissue culturing device; c) allowing the fractionated cells to adhere to a surface of the tissue culturing device; and wherein steps a) and b) of the method as defined above correspond to steps d) and e) comprising: d) culturing the adherent cells under a 5% oxygen condition; and e) passaging the adherent cells at a seeding density of 30 cells/cm²_{.}

The present invention further provides an isolated cell population derived from human bone marrow, wherein between 100% to 90% of the cells of the cell population co-express CD49c and CD90, and wherein the cell population has a doubling time of less than 30 hours, that can be obtained by any of the above methods and/or any preferred embodiments of those methods as described below.

Also described herein is a substantially homogenous cell population which co-expresses CD49c, CD90 and telomerase.

Also described herein is a substantially homogenous cell population which co-expresses CD49c and CD90, but does not express bone sialoprotein (BSP).

In another embodiment, the invention is a method as described above, wherein the cells of the cell population that co-express CD49c and CD90 do not express CD34 and/or CD45.

In a further embodiment, the invention is a method as described above, wherein the cells of the cell population which co-express CD49c and CD90 further express at least one trophic factor selected from the group consisting of BDNF, IL-6, NGF and MCP-1.

Also described herein is a method of making a substantially homogenous cell population which co-express CD49c and CD90 by culturing a source of the cell population a seeding cell density of less than about 100 cells/cm² under a low oxidative stress condition and selecting from the cultured source of the cell population, cells which co-express CD49c and CD90.

Also described herein is a method of making a substantially homogenous cell population which co-express CD49c and CD90 by culturing a source of the cell population at a seeding density of less than about 100 cells/cm² under a low oxidative stress condition; and selecting from the cultured source of the cell population, cells which co-express CD49c and CD90.

Also described herein is a method of making a substantially homogenous cell population which co-express CD49c and CD90 by culturing a source of the cell population at a seeding density of less than about 50 cells/cm² under a low oxidative stress condition; and selecting from the cultured source of the cell population, cells which co-express CD49c and CD90.

Also described herein is a method of making a substantially homogenous cell population which co-express CD49c and CD90 by culturing a source of the cell population at a seeding density of less than about 30 cells/cm² under a low oxidative stress condition; and selecting from the cultured source of the cell population, cells which co-express CD49c and CD90.

Also described herein is a method of making a substantially homogenous cell population which co-express CD49c and CD90 by culturing a source of the cell population at a seeding density of less than about 75,000 cells/cm² under a low oxidative stress condition to produce an adherent cell population and culturing the adherent cell population at a seeding density of less than about 100 cells/cm² under a low oxidative stress condition. Cells which co-express CD49c and CD90 are selected from the cultured adherent cell population.

Another embodiment of the invention includes a method of making an isolated cell population as defined above, including the step of culturing a source of the cell population at a seeding cell density of less than about 50 cells/cm² under a low oxygen condition and selecting from the cultured source of the cell population, cells which co-express CD49c and CD90.

In yet another embodiment, the invention includes a method of making an isolated cell population as defined above, including the step of culturing a source of the cell population at a seeding cell density of less than about 30 cells/cm² under a low oxygen condition and selecting from the cultured source of the cell population, cells which co-express CD49c and CD90.

In a further embodiment, the invention is a method as defined above, wherein the source of the cell population in part (a) of the method is cultured at a seeding cell density of less than 75,000 cells/cm² under a low oxygen condition of less than 15% oxygen to produce an adherent cell population; and the adherent cell population in part (b) is cultured at a seeding density of less than 100 cells/cm². Cells which co-express CD49c and CD90 are selected from the cultured adherent cell population.

Also described herein is a method of treating a human suffering from a degenerative or acute injury condition, comprising the step of administering to the human a substantially homogenous cell population which co-express CD49c and CD90.

Also described herein is a method of treating a human suffering from a neurological condition, comprising the step of administering to the human a substantially homogenous cell population which co-express CD49c and CD90.

Also described herein is a method of treating a human suffering from a cardiac condition to the human a substantially homogenous cell population which co-express CD49c and CD90.

Also described herein is a method of treating a human suffering from a neurological condition by culturing a source of a cell population at a seeding cell density of less than about 100 cells/cm² under a low oxygen condition; selecting from the cultured source of the cell population, a population of cells which co-express CD49c and CD90; and administering the population of cells which co-express CD49c and CD90 to the human.

Also described herein is a method of treating a human suffering from a neurological condition, comprising culturing a source of a cell population; selecting from the cultured source of the cell population, a population of cells which co-express CD49c and CD90; and administering the population of cells which co-express CD49c and CD90 to the human.

- Also described herein is a method of making a committed progenitor cell, comprising culturing a source of a cell population; selecting from the cultured source of the cell population, cells which co-express CD49c and CD90; and modifying the cells which co-express CD49c and CD90 to become committed progenitor cells.

Also described herein is a method of treating a human suffering from a neurological condition, comprising culturing a source of a cell population; selecting from the cultured source of the cell population, cells which co-express CD49c and CD90; modifying the cells which co-express CD49c and CD90 to become a committed progenitor cell; and administering the modified cells to the human.

Also described herein is a method of treating a human suffering from a degenerative or acute injury condition by administering to the human a substantially homogenous cell population which co-express CD49c, CD90 and telomerase.

Also described herein is a pharmaceutical composition comprising a substantially homogenous cell population which co-express CD49c and CD90.

Also described herein is a pharmaceutical composition comprising a substantially homogenous cell population which co-express CD49c, CD90 and telomerase.

Also described herein is a method of treating a human suffering from a neurological condition, comprising the step of administering to the human a substantially homogenous cell population which co-express CD49c, CD90 and telomerase.

Also described herein is a method of treating a human suffering from a degenerative or acute injury condition, comprising the step.of administering to the human a substantially homogenous cell population which co-express CD49c and CD90, but does not express bone sialoprotein (BSP).

Also described herein is a method of treating a human suffering from a neurological condition, comprising the step of administering to the human a substantially homogenous cell population which co-express CD49c and CD90, but does not express bone sialoprotein.

In still another embodiment, the invention is a method as defined above, wherein the cells of the cell population which co-express CD49c and CD90, further express at least one cardiac-related transcription factor selected from the group consisting of GATA4, Irx4 and Nkx2.5.

Also described herein is a substantially homogenous cell population which co-expresses CD49c, CD90, and at least one cardiac-related transcription factor, but does not express bone sialoprotein.

Also described herein is a substantially homogenous cell population which co-expresses CD49c, CD90, telomerase, GAT14, Irx4 and Nkx2.5.

A method of making a substantially homogenous cell population which co-expresses CD49c, CD90 and at least one cardiac-related transcription factor, may comprise the steps of culturing a source of the cell population under a low oxygen condition and treating the cultured source of the cell population with a protein kinase C inhibitor and a DNA methylation inhibitor.

Also described herein is a method of making a substantially homogenous cell population which co-expresses CD49c, CD90, telomerase and at least one cardiac-related transcription factor, comprising the steps of culturing a source of the cell population under a low oxygen condition and treating the cultured source of the cell population with the protein kinase C inhibitor and a DNA methylation inhibitor.

Also described herein is a method of making a substantially homogenous cell population which co-expresses CD49c, CD90, telomerase, GATA4, Irx4, Nkx2.5, comprising the steps of culturing a source of the cell population under a low oxygen condition and treating the cultured source of the cell population with a protein kinase C inhibitor and a DNA methylation inhibitor.

A method of making a substantially homogenous cell population which co-expresses CD49c, CD90, GATA4, Irx4, Nkx2.5, may comprise the steps of culturing a source of the cell population under a low oxygen condition and treating the cultured source of the cell population with a protein kinase C inhibitor and a DNA methylation inhibitor.

A method of making a substantially homogenous cell population which co-expresses CD49c, CD90, and at least one cardiac-related transcription factor, may comprise the step of treating a cell population which co-expresses CD49c and CD90 with a protein kinase C inhibitor and a DNA methylation inhibitor.

Also described herein is a method of making a substantially homogenous cell population which co-expresses CD49c, CD90, telomerase and at least one cardiac-related transcription factor, comprising the step of treating a cell population which co-expresses CD49c, CD90 and telomerase with a protein kinase C inhibitor and a DNA methylation inhibitor.

Also described herein is a method of making a substantially homogenous cell population which co-expresses CD49c, CD90, telomerase, GATA4, Irx4 and Nkx2.5, comprising the step of treating a cell population which co-expresses CD49c, CD90 and telomerase with a protein kinase C inhibitor and a DNA methylation inhibitor.

Also described herein is a method of treating a myocardial infarction or a congestive heart failure in a human, comprising the step of administering a substantially homogenous cell population which co-expresses CD49c, CD90 and at least one cardiac-related transcription factor to the human.

Also described herein is a method of treating a myocardial infarction or a congestive heart failure in a human, comprising the step of administering a substantially homogenous cell population which co-expresses CD49c, CD90, telomerase and at least one cardiac-related transcription factor to the human.

Also described herein is a method of treating a myocardial infarction or a congestive heart failure in a human, comprising the step of administering a substantially homogenous cell population which co-expresses CD49c, CD90, GATA4, Irx4 and Nkx2.5 to the human.

Also described herein is a method of treating a myocardial infarction or a congestive heart failure in a human, comprising the step of administering a substantially homogenous cell population which co-expresses CD49c, CD90, telomerase, GATA4, Irx4, Nkx2.5 to the human.

Also described herein is a method of treating a myocardial infarction or a congestive heart failure in an individual, comprising the steps of culturing a source of a cell population under a low oxygen condition; treating the cultured source of the cell population with a protein kinase C inhibitor and a DNA methylation inhibitor; and administering the treated cell population to the individual.

Also described herein is a method of treating a myocardial infarction or congestive heart failure in a human, comprising the steps of treating a cell population which co-expresses CD49c and CD90 with a protein kinase C inhibitor and a DNA methylation inhibitor; and administering the treated cells to the human.

Also described herein is a method of forming a committed progenitor cell-type, comprising the step of combining a substantially homogenous population of cells that co-expresses CD49c and CD90 with a population of cells that includes at least one committed progenitor cell type.

Also described herein is a pharmaceutical composition comprising a substantially homogenous cell population which co-expresses CD49c, CD90 and at least one cardiac-related transcription factor.

Also described herein is a pharmaceutical composition comprising a substantially homogenous cell population which co-expresses CD49c, CD90, telomerase and at least one cardiac-related transcription factor.

Also described herein is a pharmaceutical composition comprising a substantially homogenous cell population which co-expresses CD49c, CD90, telomerase, GATA4, Irx4, and Nkx2.5.

Also described herein is a pharmaceutical composition comprising a substantially homogenous cell population which co-expresses CD49c, CD90, GATA4, Irx4 and Nkx2.5.

The invention described herein provides a method of making an isolated population of cells as defined above, which may be used for treating a condition or disease in a human. Advantages of the cell based therapies obtainable by the methods of the claimed invention include, for example, incorporation of the cells into the tissue (e.g., central nervous system tissue, peripheral nervous system tissue, cardiac tissue); the incorporated cells have the potential to differentiate or develop into neuronal, glial or other cells (e.g., cardiac muscle) to replace or facilitate repair of the damaged, traumatized or degenerating tissue thereby resulting in a more permanent treatment of the degenerative, acute injury, traumatized, neurological or cardiac condition; and the ability to employ characterized reproducible populations of cells in treatment regimens. The cells obtained by the method of the invention have the potential to secret beneficial cytokines and trophic factors (*e.g.,* BDNF, IL-6, NGF and MCP-1).

Thus, treatment of humans with populations of cells which co-express CD49c and CD90 can potentially reverse, diminish or repair the loss due to a degenerative, acute injury, neurological (e.g., Parkinsons disease, ALS, stroke, traumatic brain injury, brain tumors) or cardiac condition (e.g., congestive heart failure, myocardial infarction) in a human, thereby increasing the quality of life and life expectancy for the human.

### BRIEF DESCRIPTION OF THE FIGURES

Figures 1A, 1B and 1C illustrate the flow cytometric analysis of cell populations in the Master and Working Cell Banks generated from a bone marrow aspirate following red blood cell lysis.
Figures 2A, 2B, and 2C illustrate the flow cytometric analysis of cell populations in the Master and Working Cell Banks generated from a bone marrow aspirate following density separation.
Figure 3 illustrates the yield (Cell Number) of cells that co-express CD49c and CD90 cells during *ex vivo* expansion of a Primary Cell Bank of colony forming units (CFUs) derived from human bone marrow aspirates.
Figure 4 illustrates the doubling rate of cell populations that co-express CD49c and CD90 cell population in culture.
Figure 5 illustrates the Basso-Beattie-Bresnahan (BBB) index for rats following spinal cord injury and after transplantation with a substantially homogenous cell population which co-express CD49c and CD90. At 19 days post contusion, rat received the cell transplantation showed greater improvement than rat received only PBS control.
Figure 6A depicts the effects of early-myocytic determined cells (EMD) and unmodified cells on cardiac function following experimental induction of myocardial infarction as measured by peak positive rate of pressure change (+dp/dt). Four weeks after treatment, rats receiving either unmodified cells or early-myocytic determined cells had significantly higher +dp/dt values. (*p<0.05 by ANOVA).
Figure 6B depicts gender differences in male and female rats following experimental induction of myocardial infarction and treatment with early-myocytic determined cells (EMD) and unmodified cells. (*p<0.05 by ANOVA).
Figure 6C depicts changes in cardiac function over the course of treatment by subtracting 0 week +dp/dt values from 4 week +dp/dt values to derive a "delta +dp/dt" in rats following experimental induction of myocardial infarction and treatment with early-myocytic determined cells (EMD) or unmodified cells. (*p<0.05, **p<0.01 by ANOVA).
Figure 6D depicts gender differences in male and female rats following experimental induction of myocardial infarction and treatment with early-myocytic determined cells (EMD) and unmodified cells. (*p<0.05, **p<0.01 by ANOVA).
Figures 7A and 7B depict the effects of treatment with early-myocytic determined cells (EMD) and unmodified cells on cardiac function in rats following experimental induction of myocardial infarction as measured by peak negative rate of pressure change (-dp/dt). (*p<0.05, **p<0.01 by ANOVA).
Figures 8A and 8B depicts the effects of unmodified cells and early-myocytic determined cells (EMD) on cardiac function in rats following experimental induction of myocardial infarction as measured by the time constant of isovolumetric left ventricular pressure decay (tau). (*p<0.05, **p<0.01 by ANOVA).
Figure 9 depicts the histological score (fibrotic/viable tissue area) in cardiac muscle obtained from rats following experimental induction of myocaridal infarction and subsequent treatment with unmodified cells or early-myocytic determined cells (EMD).
Figures 10A and 10B are H & E and trichrome staining of cardiac tissue sections obtained from rats following experimental induction of myocardial infarction and subsequent treatment with vehicle (Figure 10A) or unmodified cells (Figure 10B). The white line depicts the area of the myocardial infarction.

### DETAILED DESCRIPTION OF THE INVENTION

The features and other details of the invention, either as steps of the invention or as combinations of parts of the invention, will now be more particularly described and pointed out in the claims. It will be understood that the particular embodiments of the invention are shown by way of illustration and not as limitations of the invention. The principle features of this invention can be employed in various embodiments without departing from the scope of the invention.

The present invention relates to a method of making an isolated cell population derived from human bone marrow, wherein between 100% to 90% of the cells of the cell population co-express CD49c and CD90, and wherein the cell population has a doubling time of less than 30 hours, comprising the steps of:
a) culturing a source of the cell population under a low oxygen condition of less than 15% oxygen to produce an adherent cell population; and,
b) culturing the adherent cell population at a seeding density of less than 2500 cells/cm². The invention also relates to the method as defined above, wherein the cells of the population which co-express CD49c, and CD90, further express at least one cardiac-related transcription factor (*e.g*., GATA4, Irx4 and Nkx2.5).

"Substantially homogenous" as used herein refers to a population of cells wherein the majority (e.g., between about 100% to about 90%) of the total number of cells have a specified characteristic of interest (e.g., co-express CD49c and CD90; co-express CD49c and CD90 with minimal expression of CD34 and/or CD45).

"Co-express" or "co-expresses," as used herein, refers to the simultaneous detection of two or more molecules, e.g., CD49c and CD90; CD49c, CD90 and telomerase; CD49c, CD90, GATA4, Nkx2.5 and Irx4; CD49c, CD90, telomerase, GATA4, Nkx2.5 and Irx4, on or in a single cell; or, alternatively, on or in a collection of cells.

The substantially homogenous cell population of the invention co-expresss CD49c and CD90 on a single cell of the population. The cell population of the invention which co-expresses CD49c, CD90 and, optionally, telomerase on each cell of the population, can also co-express other proteins (e.g., cardiac-related transcription factors such as GATA4, Irx4 and Nkx2.5) on at least a portion (e.g., about 10%, about 20%, about 50%, about 70%, about 80%, about 90%) of the total number of cells in the cell population.

Techniques to detect co-expression of CD49c and CD90 in cells (e.g., bone marrow stromal cells) are well established. For example, co-expression of CD49c and CD90 on a cell can be detected by multiple color cytometric analysis. CD49c can be detected employing a fluorescein labeled probe and CD90 can be detected employing a Texas red probe. The CD49c and CD90 cell surface antigens can be visualized with the aid of a flow cytometer equipped with multiple filters capable of detecting the multiple colors. Techniques to detect the molecules of interest can also include ELISA, RIA, immunoflouresence microscopy and quantitative PCR.

Also described herein is a substantially homogenous cell population of the invention which co-express CD49c and CD90, but does not express bone sialoprotein.

The isolated cell population produced according to the method of the invention which co-express CD49c and CD90, has a doubling time of less than 30 hours. The doubling time of the cells of the invention can be varied depending on, for example, the density of the cells in culture (e.g., 100 cells/cm²) and/or the concentration of oxygen employed to culture the cells (e.g., a low oxygen concentration such as about 5% oxygen).

The substantially homogenous cell population which co-express CD49c and CD90 can have the potential to differentiate into a preselected phenotype (e.g., chondrocytes, astrocytes, oligodendrocytes, neurons, bone, osteoclasts, osteoblasts, cardiomyocytes, pancreatic islet cells, skeletal muscle, smooth muscle, hepatocytes and retinal ganglial cells). The potential to differentiate into a preselected phenotype refers to the ability of the cell population to change to a functional cell type.

The substantially homogenous cell population which co-express CD49c and CD90 do not, after between about 20 population doublings to about 50 population doublings, substantially express at least one cell senescent marker selected from a group consisting of P21 and P53. A senescent marker would be any marker associated with senescence or aging in a cell (e.g., P21, P53). The senescent marker can be a cytoplasmic, nuclear or cell surface marker.

In one embodiment, the isolated cell population produced according to the method has been cultured *in vitro* through a number of population doublings selected from the group consisting of a) at least 20 population doublings; b) at least 30 population doublings; c) at least 40 population doublings; and d) at least 50 population doublings; and still co-express CD49c and CD90 but does not substantially express at least one cell senescent marker selected from a group consisting of P21 and P53. One of skill in the art would be able to determine when a cell has undergone a population doubling (Freshney, R.I. "Culture of Animal Cells: A Manual of Basic Techniques" New York, Wiley-Liss (1994)) and be able to determine whether the cell populations co-express CD49c and CD90 and do not substantially express at least one cell senescent marker selected from a group consisting of P21 and P53 employing established techniques (e.g., flow cytometry, quantitative PCR).

The isolated cell population obtained by the method of the invention which co-express CD49c and CD90 can further include expression of P21 or P53 after between about 20 to about 50 population doublings of the cells. Expression of a senescent marker (e.g., P21, P53) is a relative expression of the senescent marker (e.g., relative to 18s rRNA GAPDH (Glyceraldehyde-3-phosphate dehydrogenase), actin). "Relative expression," as used herein, is expression (e.g., nucleic acid, protein) of a molecule of interest (e.g., CD49c, CD90, telomerase, CBFA1, BSP, BDNF, IL-6, MCP-1) with respect to expression of a standard or reference marker (e.g. 18s rRNA, actin, GFAP). In a preferred embodiment, expression of P53 is a relative expression of up to about 3000 transcripts of P53 (e.g., 0, 100, 1000, 1500, 2000) per 10⁶ transcripts of an 18s rRNA and expression of P21 is a relative expression of up to about 20,000 transcripts of P21 per 10⁶ transcripts of an 18s rRNA.

In one embodiment, the expression of p53 is about 3000 transcripts of p53 per 10⁶ transcripts of an 18s rRNA. In another embodiment, the expression of p53 is about 2000 transcripts of p53 per 10⁶ transcripts of an 18s rRNA. In yet another embodiment, the expression of p53 is about 1000 transcripts of p53 per 10⁶ transcripts of an 18s rRNA.

In another embodiment, the expression of p21 is up to about 20,000 transcripts of p21 (e.g., 0, 100, 1000, 5000, 10000, 15000, 20000) per 10⁶ transcripts of an 18s rRNA. In still another embodiment, the expression of p21 is about 15,000 transcripts of p21 per 10⁶ transcripts of an 18s rRNA. In yet another embodiment, the expression of p21 is about 500 transcripts of p21 per 10⁶ transcripts of an 18s rRNA.

In one embodiment, the expression of a bone lineage marker core binding factor 1 (CBFA1) (Otto, F. et al., Cell 89(5) 765-771 (1997)) is about 5000 transcripts of the bone lineage marker per 10⁶ transcripts of an 18s rRNA. In another embodiment, the expression of the bone lineage marker CBFA1 is about 3000 transcripts of the bone lineage marker per 10⁶ transcripts of an 18s rRNA. In still another embodiment, the expression of the bone lineage marker CBFA1 is about 1000 transcripts of the bone lineage marker per 10⁶ transcripts of an 18s rRNA.

The cell population produced according to the method of the invention is a cell population from human bone marrow. Thus, the substantially homogenous cell population is derived from human bone marrow cells (e.g., human bone marrow stromal cells). Cells of the invention can be referred to as "derived" from human bone marrow. Cells derived from human bone marrow can be obtained, for example, by lysis of the source of the cells (e.g. bone marrow cells). For example, bone marrow stromal cells are derived from whole bone marrow aspirates after ammonium chloride lysis of the bone marrow aspirates. The ammonium chloride removes red blood cells from the aspirates and the resulting cell pellet is employed to generate the cell population which co-express CD49c and CD90 cells according to the method of the invention. Alternatively, the bone marrow can be processed (e.g., fractionated by density gradient centrifugation, NH₂Cl lysis, fluorescent activated sorting or magnetic sorting to derive the source of the cell population to be used in the methods of the invention. For example, the bone marrow aspirates or lysed bone marrow cells are passed through a density gradient to separate the cells of the invention from cellular debris as a result of lysis. Alternatively, or additionally, the bone marrow aspirates or lysed bone marrow cells can form a density gradient.

Whole bone marrow aspirates are obtained from a human and cultured in contact with a solid phase. Alternatively, or additionally, the whole bone marrow aspirate can be processed to yield a mononuclear cell fraction which is then cultured in contact with a solid phase. The solid phase can be, for example, plastic (e.g., tissue culture treated plastics).

The mononuclear cell fraction can be obtained from a whole bone marrow aspirate on a density gradient by established procedures. Alternatively, the mononuclear cell fraction is obtained by lysis of the red blood cells contained in the bone marrow aspirate. The lysis is done by mixing the bone marrow aspirate with ammonium chloride.

Human bone marrow cells are obtained from healthy human donors by aspirations of the iliac crest and bone marrow stromal cell populations obtained employing well-established techniques. For example, isolated cell populations of the invention which co-express CD49c and CD90 are obtained from human iliac crest bone marrow aspirates and processed to mononuclear cell fractions from which bone marrow stromal cells are selectively propagated *in vitro* based upon their propensity to attach to plastic and divide in response to defined cell culture medium. The plastic-adherent cells are optimally grown at a cell concentration that encourages virtually only the self-renewing cells, referred to as colony-forming unit fibroblast-like cells (Cfu-f), to proliferate. The Cfu-f-derived cells are analyzed for cells which co-express CD49c and CD90 and sub-cultured according to the method of the invention to produce a cell population which co-express CD49c and CD90.

The bone marrow aspirate, or a cellular fraction of the bone marrow aspirate, is cultured in contact with a solid phase and an intermediate cell population is isolated from the resulting cell culture based on their propensity to adhere to the solid phase. Bone marrow aspirates, or a cellular fraction of the aspirate, are cultured according to part (b) of the method of the invention at a dissolved oxygen concentration of less than 20%, preferably between 1% to about 10%, and most preferably from between 2% oxygen to 7% oxygen. In a preferred embodiment, the dissolved oxygen concentration is 5% oxygen. The resulting adherent cell population is expanded to yield isolated cell population which co-express CD49c and CD90.

Bone marrow cell expansion according to part (b) of the method of the invention is conducted with a seeding density of less than 2500 cells/cm², preferably less than 1000 cells/cm², and most preferably less than 100 cells/cm². In a particular embodiment, the initial cell density in the expansion step is between 30 cells/cm² to 50 cells/cm². A seeding density would be the number of adherent cells cm² obtained from mononuclear bone marrow cells.

Standard media preparations can be used to culture the bone marrow cells. For example, the media can be minimum essential medium-alpha modification supplemented with 4mM L-glutamine and 0 to 10% lot selected fetal bovine serum (FSB), preferably about 10% FSB. The culturing step can be conducted for any reasonable period, for example, between about 3 to about 25 days and most preferably between about 3 to about 15 days.

An intermediate cell population is isolated from the cell culture describe above based on its propensity to adhere to the solid phase. The intermediate cell population is grown at a cell concentration that encourages virtually only the self-renewing cells, referred to herein as colony-forming unit fibroblast-like cells (Cfu-f), to proliferate. The Cfu-f-derived cells are sub-cultured under defined conditions to produce a substantially homogeneous population of cells (Example 1). According to the invention, the expansion yields a substantially homogeneous cell population which co-express CD 49 and CD 90.

In another embodiment, cells of the cell population produced according to the method of the invention do not express CD34 and/or CD45. The presence or absence of CD34 and CD45 can be detected on bone marrow mononuclear cells which co-express CD49c and CD90 using routine methods including, for example, antigen-specific ELISA assays, quantitative PCR, or flow cytometry. Cells which co-express CD49c and CD90, but

do not express either or both CD34 and/or CD45, are propagated in culture and stored until use in the methods described herein.

In yet another embodiment, the cells of the cell population produced according to the method of the invention co-expressing CD49c and CD90 further express at least one trophic factor selected from the group consisting of brain-derived neurotrophic factor (BDNF) (Barde, Y.A., et al. EMBO J., 1(5):549-553 (1982)), nerve growth factor (NGF) (Levi-Montalcini, R., Arch Biol 76(2):387-417 (1965)), neurotrophin-3 (NT-3) (Mohn, A., et al., Nature 344:339-341 (1990)), interleukin-6 (IL-6) (Barton, B.E., Clin. Immumol. Immunopathol. 85(1):16-20 (1997)), interleukin-7 (IL-7), interleukin-11 (IL-11), stem cell factor (SCF), macrophage chemoattractin protein-1 (MCP-1), matrix metalloproteinase-9 (MMP-9) and Cystatin-C and vascular endothelial growth factor (VEGF) (Moore, MA., et al., Ann. N.Y. Acad. Sci., 938:36-45 (2001); Kollermann, J., et al., Am. J. Clin. Pathol. 116:115-121 (2002)).

Expression of BDNF, NGF, NT-3, IL-6, IL-7, IL-11, SCF, VEGF, MCP-1, MMP-9 and Cystatin-C in populations of cells co-expressing CD49c and CD90 can be augmented by a variety of techniques, including *ex vivo* cultivation of the cells in chemically defined medium.

- Also described herein is a substantially homogenous cell population which co-express CD49c, CD90 and telomerase. Expression of telomerase is a relative expression, for example, a relative expression of greater than between about 1 transcript of telomerase per 10⁶ transcripts of an 18s rRNA and about 10 transcripts of telomerase per 10⁶ transcripts of an 18s rRNA.

Expression of telomerase is about 1 transcript of telomerase per 10⁶ transcripts of an 18s rRNA. In another embodiment, expression of telomerase is about 5 transcripts of telomerase per 10⁶ transcripts of an 18s rRNA.

Expression of telomerase is about 10 transcripts of telomerase per 10⁶ transcripts of an 18s rRNA.

The cell population which co-expresses CD49c, CD90 and telomerase has a doubling time of less than about 144 hours, less than about 72 hours or less than about 48 hours.

The cell population which co-expresses CD49c, CD90 and telomerase has the potential to differentiate into a preselected phenotypes (e.g., a chondrocyte, an astrocyte, an oligodendrocyte, a neuron, osteocyte, osteoblast, osteoclast, a cardiomyocyte, a pancreatic islet cell, a skeletal muscle, a smooth muscle, a hepatocyte and a retinal ganglial cell).

The cell population which co-expresses CD49c, CD90 and telomerase can further include expression of P21 or P53 after between about 20 to about 50 population doublings of the cells (e.g., 20, 30, 40 or 50 population doublings). Expression of P53 is a relative expression of up to about 3000 transcripts of P53 per 10⁶ transcripts of an 18s rRNA (e.g, 3000, 2000 or 1000 transcripts of P53 per 10⁶ transcripts of an 18s RNA). Expression of P21 is a relative expression of up to about 20,000 transcripts of P21 per 10⁶ transcripts of an 18s rRNA (e.g., 20000, 15000 or 5000 transcripts of P21 per 106 transcripts of an 18s rRNA).

The cell population which co-expresses CD49c, CD90 and telomerase does not express CD34 and/or CD45.

The cell population which co-expresses CD49c, CD90 and telomerase may express at least one trophic factor selected from the group consisting of BDNF, IL-6 andMCP-1.

In one embodiment, the invention includes a method of making an isolated cell population as defined above comprising culturing a source of the cell population (e.g., human bone marrow cells) and selecting from the cultured source of the cell population, cells which co-express CD49c and CD90. The source of the cell population is cultured under a low oxygen condition of less than 15% oxygen (e.g., less than atmospheric). "Low oxygen condition," as used herein, refers to a concentration (e.g., percent of oxygen based on volume, weight or molarity) of less than 15% oxygen, which is less than atmospheric oxygen.

Also described herein is a method of making a substantially homogenous cell population which co-express CD49c, CD90 and telomerase comprising culturing a source of the cell population (e.g., human bone marrow cells) and selecting from the cultured source of the cell population, cells which co-express CD49c, CD90 and telomerase.

Also described herein is a method of making a substantially homogenous cell population which co-express CD49c and CD90 but does not express bone salioprotein comprising culturing a source of the cell population and selecting from the cultured source of the cell population, cells which co-express CD49c, CD90 and a bone lineage marker.

The low oxygen condition used in part (a) of the method of the invention is an oxygen concentration of less than 15% by volume (mole percent) oxygen, and more preferably an oxygen concentration less than 10% by volume, and most preferably an oxygen concentration is less than 5% by volume oxygen. In another embodiment, the source of the cell population is cultured at a seeding density of less than about 100 cells/cm² (e.g., 95, 90, 80, 50, 30, 25 cells/cm²) under low oxygen conditions of less than 15% oxygen (e.g., less than about 5% oxygen).

Also described herein is a method of making a substantially homogenous cell population which co-expresses CD49c and CD90 comprising culturing a source of the cell population (e.g., human bone marrow cells) and selecting from the cultured source of the cell population, cells which co-express CD40c and CD90 by culturing the source of the cell population under low oxidative stress (e.g., glutathione, Vitamin C, Catalase, Vitamin E, N-Acetylacysteine). "Low oxidative stress," as used herein, refers to conditions of no or minimal free radical damage to the cultured cells.

The method according to the invention can further include lysing the source of the cell population (e.g., bone marrow aspirates) prior to culturing the source of the cell population. For example, lysis of a bone marrow aspirate can result in the lysis of hematopoietic cells leaving the non-hematopoietic cells un-lysed. Additionally, or alternatively, the method of the invention can further include fractionating (e.g., by passage through or formation of a density gradient, by NH₂Cl

lysis) the source of the cell population (e.g., bone marrow aspirates) prior to culturing the source of the cell population. Thus, the method according to the invention may further include selecting a fractionated source of the cell population prior to culturing the source of the cell population.

The cells made by the method of the invention can also express at least one trophic factor (e.g., BDNF, NGF, NT-3, IL-6, IL-7, IL-11, SCF, MCP-1, MMP-9, Cystatin-C and VEGF). In another embodiment, the substantially homogenous population of cells which co-express CD49c and CD90; made by the method of the invention do not express CD34 and/or CD45.

Also described herein is a method of treating a human suffering from a degenerative or acute injury condition, comprising the step of administering to the human a substantially homogenous cell population which co-expresses CD49c and CD90. The cells used to treat the human suffering from a degenerative or acute injury condition can also not express CD34 and/or CD45.

Degenerative disease is a disease in which the decline (e.g., function, structure, biochemistry) of particular cell type (e.g., neuronal, muscle, connective, epithelial) results in an adverse clinical condition. For example, Parkinson's disease is a degenerative disease in the central nervous system (e.g., basal ganglia) which is characterized by rhythmical muscular tremors, rigidity of movement, festination, droopy posture and masklike facies. Degenerative diseases that can be treated with the isolated cell population obtained by the methods of the invention which co-express CD49c and CD90 can be, for example, Parkinson's disease, Huntington's disease, Alzheimer's disease, amyotrophic lateral sclerosis, congenital heart failure, cardiomyopathy, ataxias, and spinal muscular dystrophy.

An acute injury condition is a condition in which an event or multiple events results in an adverse clinical condition. The event which results in the acute injury condition can be an external event such as blunt force or compression or an internal event such as sudden ischemia (e.g., stroke or heart attack). Acute injury conditions that can be treated with the isolated cell populations obtained by the methods of the invention which co-express CD49c and CD90; can be, for example, spinal cord injury, traumatic brain injury, myocardial infarction, congestive heart failure and stroke.

Also described herein is a method of treating a human suffering from a cardiac condition, comprising the step of administering to the human a substantially homogenous cell population which co-express CD49c and CD90. A cardiac condition is a disease of the heart. The disease of the heart can be a disease of the cardiac muscle, connective tissue of vessels of the heart. The cells used to treat the human suffering from a cardiac condition can also not express CD34 and/or CD45. A cardiac condition that can be treated by the cells obtained by the methods of the invention can be, for example, myocardial infarction, congestive heart failure, myocarditis, vascular heart disease, cardiomyopathy, congenital heart disease, eschemic heart disease, heart transplant and pre-transplantation bridge.

Also described herein is a method of treating a human suffering from a neurological condition, comprising the step of administering to the human a substantially homogenous cell population which co-express CD49c and CD90; co-express CD49c, CD90 and telomerase; CD49c, CD90 and a bone lineage marker. "A neurological condition," as used herein, refers to any state of the nervous system (central or peripheral nervous system) which deviates in any manner from a normal nervous system or nervous system of a mammal (e.g., human) not affected by a neurological condition. The neurological condition can be a condition of the central (brain or spinal cord) or peripheral nervous system. The neurological condition can be, for example, the result or consequence of a disease (e.g., amyotrophic lateral sclerosis, Parkinson's Disease, Fabry Disease), acute injury condition (e.g., stroke, brain injury, spinal cord injury) or a combination of disease and acute injury condition. Other neurological conditions which can be treated with the isolated cell population obtained by the method of the invention include, for example, metachromatic distropy, adrenal leukodystrophy, Canavan disease, Pelizaeus Merzbacher, Nieman-pick and a brain tumor.

Also described herein is a method of treating a human suffering from a neurological condition, comprising culturing (e.g., low oxygen conditions; oxygen conditions less than atmospheric; about 5% oxygen) a source of a cell population (e.g., bone marrow, fat, cord blood, skin) and selecting from the cultured source of the cell population, a population of cells which co-express CD49c and CD90. The selected population of cells which co-express CD49c and CD90 are administered to the human.

The substantially homogenous population of cells which are administered to the human may co-express CD49c and CD90 and lack CD34 and/or CD45. The substantially homogenous population of cells which are administered to the human may co-express CD49c, CD90 and telomerase. The substantially homogenous population of cells which are administered to the human may co-express CD49c and CD90 or co-express CD49c, CD90 and telomerase and express at least three trophic factors selected from the group consisting of BDNF, NGF, NT-3, IL-6, IL-7, IL-11, SCF, MCP-1, MMP-9 and Cystatin-C (e.g., BDNF, IL-6 and MCP-1).

The synthesis and secretion of cytokines and trophic factors from the isolated population of cells obtained by the methods of the invention can protect surrounding cells near or distant from the site of transplantation from further damage as a consequence of the degenerative, acute injury or neurological condition. The synthesis and secretion of cytolunes and trophic factors from the isolated population of cells obtained by the methods of the invention can also, or alternatively, promote regeneration of cells and tissues of the host (e.g., human suffering from a acute injury, neurological, cardiac or degenerative condition) treated with the isolated population cells which co-express CD49c and CD90.

The isolated population of cells which co-express CD49c and CD90 obtained by the methods of the invention when administered to the human may respond to cellular signaling and physiological cues in the human and migrate to the area of injury or trauma and, therefore, be used as delivery vehicles for proteins and genes of interest.

Also described herein is a method of treating a human suffering from a neurological condition (e.g., spinal cord injury, an amyotrophic lateral sclerosis, a Parkinson's Disease, a stroke, a traumatic brain injury, a Fabry Disease condition, metachromatic distropy, adrenal leukodystrophy, Canavan disease, Pelizaeus Merzbacher, Nieman-pick, a brain tumor) by culturing a source of a cell population at a seeding density of less than about 100 cells/cm² under a low oxygen condition; selecting from the cultured source of the cell population, a population of cells which co-express CD49c and CD90; and administering the population of cells which co-express CD49c and CD90 to the human.

The transplantation of the isolated cell populations obtained by the methods of the invention into a patient suffering from a neurological condition may result in the differentiation of the cells of the invention into cells which normally function in the nervous tissue affected in the human with the neurological condition thereby treating a myriad of neurological conditions including, for example, Parkinson's disease, ALS, spinal cord injury, brain tumors, stroke. Similarly, the isolated cell populations obtained by the methods of the invention can be used to treat a human suffering from a non-neurological condition such as a burn, heart disease, diabetes, osteoarthritis and rheumatoid arthritis. The isolated cell populations obtained by the methods of the invention administered to a patient (also referred to herein as an individual, in a particular human) suffering from a cardiac condition (e.g., myocardial infarction) can differentiate into cardiac muscle cells (also referred to herein as cardiomyocytes).

The cell populations obtained by the methods of the invention may have the capacity to respond to intrinsic signals (e.g., at the sites of transplantation or when incorporated into tissues and organs) and exogenous cues to differentiate into numerous cell types (e.g., neuronal, glial, astrocytes, oligodendrocystes) in the human. The cell populations obtained by the methods of the invention can provide a readily available source of cells for use in treating humans. The cell populations obtained by the methods of the invention can be readily isolated from adult or embryonic tissues, proliferate at high rates, have large expansion potential, can be stable for long periods of time, can be responsive to exogenous signals and can produce sufficient therapeutic quantities of molecules of interest.

Accordingly, also described herein is a method of making a committed progenitor cell by culturing (e.g., under a low oxygen condition, 5% oxygen) a source of a cell population (e.g., bone marrow cells, human bone marrow cells, fat, cord blood, skin) and selecting from the cultured source of the cell population cells which co-express CD49c and CD90. The population of cells which co-express CD49c and CD90 are modified to become committed progenitor cells. The selection of cells from the cultured source of the cell population cells which co-express CD49c and CD90 is achieved by a low oxygen condition (e.g., oxygen below atmospheric oxygen, 5% oxygen).

"Committed progenitor cell," as used herein, refers to a precursor cell obtained from a source (e.g., human bone marrow, fat, cord blood, skin) which develops into a cell for a particular purpose. A committed progenitor cell can be, for example, a CD49c/CD90 cell derived from human bone marrow which can differentiate or develop into, for example, a neuron, glial, astrocyte, oligodendrocyte cell or cardiac muscle cell.

Also described herein is a method of treating a human suffering from a neurological condition by culturing a source of a cell population (e.g., bone marrow aspirates) and selecting (e.g., by a low oxygen culture condition) from the cultured source of the cell population, cells which co-express CD49c and CD90; co-express CD49c, CD90 and telomerase; or co-express CD49c, CD90 and a bone lineage marker. The selected cells which co-express, for example, CD49c and CD90 are modified to become a committed progenitor cell and administered to a human with a neurological condition (e.g., a spinal cord injury, an amyotrophic lateral sclerosis, a Parkinson's Disease, a stroke, a traumatic brain injury, a Fabry Disease condition, metachromatic distropy, adrenal leukodystrophy, Canavan disease, Pelizaeus Merzbacher, Nieman-pick and a brain tumor).

Techniques to assess whether a cell of the substantially homogenous population of cells of the invention which co-express CD49c and CD90; co-express CD49c, CD90 and telomerase; or CD49c, CD90 but does not express bone salioprotein (BSP) become committed progenitor cells are within the expertise of one of skill in the art. For example, cells which co-express CD49c and CD90 can be cultured, selected and modified to produce and express the neuronal cell markers, such as noggin, musashi or Sox2, which would indicate that the cells are committed neuronal progenitors cells. Techniques to determine whether a cell has become a committed progenitor cell are well-established and known to one of skill in the art (e.g., quantitative PCR, flow cytometry).

Cells which co-express CD49c and CD90 can be selected from a source of a cell population for making the committed progenitor cells. Selected cells which co-express CD49c and CD90 (also referred to herein as "selected cells") can be, for example, modified to become committed progenitor cells by culturing the selected cells in:
1. DMEM/F12/ITS/2mM GlutamineBSAlmg/ml;
2. DMEM/F12/ITS/2mM Glutamine/BSAlmg/ml/0.25ng/ml IL-β;
3. DMEM/F12/ITS/2mM Glutamine/BSAlmg/ml/2ng/ml TNFα;
4. DMEM/F12/ITS/2mM Glutamine/BSA1mg/ml/100ng/ml NT-3;
5. DMEM/F12/ITS/2mM Glutamine/BSA1mg/ml/100ng/ml Noggin;
6. DMEM/F12/ITS/2mM Glutamine/BSA1mg/ml/100ng/ml GDNF;
7. DMEM/F12/ITS/2mM Glutamine/BSA1mg/ml/20ng/ml/ bFGF;
8. DMEM/F12/ITS/2mM Glutamine/BSA1mg/ml/10µM Forskolin;
9. DMEM/F12/ITS/2mM Glutamine/BSA1mg/ml/1µM Bay K 8644; and/or
10. DMEM/F12/B27/2mM Glutamine/BSA1mg/ml.
11. MEM-Alpha/4mM Glutamine/10% ser lot selected fetal bovine serum and 5mM nifedipine

Selected cells can be used directly from cultures or stored for future use (e.g., by freezing in liquid nitrogen).

The committed progenitor cells described herein may not express CD34 and/or CD45. The committed progenitor cells described herein may express at least one trophic factor selected from the group consisting ofBDNF, NGF, NT-3, IL-6, IL-7, IL-11, SCF, MCP-1, VEGF, matrix metalloproteinase-9 (MMP-9) and Cystatin-C (e.g., BDNF, lL-6 and MCP-1).

Also described herein is a method of treating a human suffering from a neurological condition, comprising culturing a source of a cell population (e.g., bone marrow, human bone marrow, fat, cord blood, skin) and selecting from the cultured source of the cell population cells which co-express CD49c and CD90. The selected cells are modified to become a committed progenitor cell. The committed progenitor cells are administered to a human.

Also described herein is a substantially homogenous cell population which co-expresses CD49c, CD90 and at least one cardiac-related transcription factor. The cell population which co-expresses CD49c, CD90 and at least one cardiac-related transcription factor can further include expression of telomerase. The substantially homogenous cell population which co-expresses CD49c, CD90 and at least one cardiac-related transcription factor may be derived from human bone marrow cells.

"Cardiac-related transcription factor," as used herein, refers to a protein, or the gene encoding a protein, or portion of a protein, which regulates the transcription of genes associated with or expressed by cardiac muscle cells. In a particular embodiment, the cardiac-related transcription factor is selected from the group consisting of GATA-binding transcription factor 4 (GATA4) (Auda-Boucher, G. et al., Dev. Bio. 225:214-225 (2000)); Iroquois Homeobox Gene 4 (Irx4) (Bao, Z., et al., Science 283:1161-1164 (1999); Auda-Boucher, G., et al., Dev. Bio. 225:214-225 (2000)); and Nkx2.5/CSX (Cardio specific Homeobox), also referred to as "Nkx2.5," (Bao, Z., et al., Science 283:1161-1164 (1999); Auda-Boucher, G., et al., Dev. Bio. 225:214-225 (2000)).

The substantially homogenous cell population which co-expresses CD49c, CD90, at least one cardiac-related transcription factor, and, optionally, telomerase, can differentiate into a cardiac muscle cell (also referred to herein as a cardiomyocyte). The substantially homogenous cell population which co-expresses CD49c, CD90, at least one cardiac-related transcription factor, and, optionally, telomerase, can also express at least one trophic factor (e.g., IL-6, VEGF, MCP1, BDMF).

Also described herein is a substantially homogenous cell population in which co-expresses CD49c, CD90 and at least one cardiac-related transcription factor, but does not express bone sialoprotein. The substantially homogenous cell population, which does not express bone sialoprotein, can also express a cardiac-related transcription factor (e.g., GATA4, Irx4, Nkx2.5).

Also described herein is a substantially homogenous cell population which co-expresses CD49c, CD90, and at least one member selected from the group consisting of GATA4, Irx4 and Nkx2.5. This substantially homogenous cell population can further express telomerase.

- Also described herein is a method of making a substantially homogenous cell population which co-expresses CD49c, CD90 and at least one cardiac-related transcription factor comprising culturing a source of the cell population under a low oxygen condition and treating the cultured source of the cell population with the protein kinase C inhibitor (e.g., chelerythrine, H-7 dehydrochloride, K252a, staurosporine, bisindolylmaleimide I-V and Calphostin C) and a DNA methylation inhibitor (e.g, azacytidine). The method can further include selecting from the treated cell population cells which co-express CD49c, CD90 and at least one cardiac-related transcription factor.

The source of the cell population used in the methods of the invention includes a human bone marrow source. In a more preferred embodiment, the human bone marrow source is an adult bone marrow source.

In a particular embodiment, the protein kinase C inhibitor used in the methods of the invention is chelerythrine and the DNA methylation inhibitor is 5-azacytidine.

Also described herein is a method of making a substantially homogenous cell population which co-expresses CD49c, CD90, telomerase and at least one cardiac-related transcription factor comprising the steps of culturing a source of the cell population under a low oxygen condition and treating the cultured source of the cell population with a protein kinase C inhibitor and a DNA methylation inhibitor.

The isolated populations obtained by the methods of the invention which co-express CD49c, CD90 and at least one cardiac-related transcription factor have longer doubling times (e.g., less than about 72 hours, less than about 65 hours, less than about 60 hours) than cells of the invention which were not treated with a protein kinase C inhibitor and a DNA methylation inhibitor and do not express at least one cardiac-related transcription factor. The cell populations obtained by the methods of the invention can be labeled. Labeled cells can be administered to individuals (e.g., laboratory animals, such as rats, mice, or humans) and the fate of the labeled cells determined at a time (e.g., days, months, years) after administration. The cells can be radiolabeled, flourescently labeled, or labeled with other compounds (e.g., biotin) to permit localization in the individual.

Also described herein is a method of making a substantially homogenous cell population which co-expresses CD49c, CD90, telomerase and at least one cardiac-related transcription factor, comprising the steps of culturing a source of the cell population (e.g., adult bone marrow cells) under a low oxygen condition and treating the cultured source of the cell population with a protein kinase C inhibitor (e.g., chelerythrine) and a DNA methylation inhibitor (e.g., 5-azacytidine).

Also described herein is a method of making a substantially homogenous cell population which co-expresses CD49c, CD90, telomerase, GATA4, Irx4 and Nkx2.5, comprising the steps of culturing a source of a cell population under a low oxygen condition and treating the cultured source of the cell population with a protein kinase C inhibitor and a DNA methylation inhibitor. The method further includes selecting from the treated cell population, cells which co-express CD49c, CD90, telomerase, and at least one member selected from the group consisting of GATA4, Irx4 and Nkx2.5.

Also described herein is the method of making a substantially homogenous cell population which co-expresses CD49c, CD90, GATA4, Irx4, and Nkx2.5, comprising the steps of culturing a source of a cell population under low oxygen conditions and treating the cultured source of the cell population with a protein kinase C inhibitor in a DNA methylation inhibitor. Cells which co-express CD49c, CD90, telomerase, GATA4, Irx4 and Nix2.5 can be selected from the treated cells.

Also described herein is a method of making a substantially homogenous cell population which co-expresses CD49c, CD90 and at least one cardiac-related transcription factor, comprising the step of treating a cell population which co-expresses CD49c and CD90 with a protein kinase C inhibitor and a DNA methylation inhibitor.

Also described herein is a method of making a substantially homogenous cell population which co-expresses CD49c, CD90, telomerase and at least one cardiac-related transcription factor, comprising the step of treating a cell population which co-expresses CD49c, CD90 and telomerase with a protein kinase C inhibitor and a DNA methylation inhibitor.

Also described herein is a method of making a substantially homogenous cell population which co-expresses CD49c, CD90, telomerase, GATA4, Irx4 and Nkx2.5, comprising the step of treating a cell population which co-expresses CD49c, CD90 and telomerase with a protein kinase C inhibitor and a DNA methylation inhibitor.

The methods of the invention can further include selecting from the treated cells, cells which co-express CD49c, CD90 and at least one cardiac-related transcription factor (e.g., GATA4, Irx4, Nkx2.5)

In one embodiment, the cell population of the invention can be a stem cell population. In another embodiment, the cell population of the invention can be an isolated cell population or an isolated stem cell population.

Cell populations obtained by the methods of the invention, in particular, cell populations which co-express at least one cardiac-related transcription factor can be used to treat a cardiac condition such as myocardial infarction or congestive heart failure in a human. Cell populations find application in a method of treating a myocardial infarction or congestive heart failure in an individual (e.g., a human), comprising the step of administering a substantially homogenous cell population which co-express CD49c, CD90, at least one cardiac-related transcription factor to the individual.

The, cardiac-related transcription factor expressed by the cells used to treat the myocardial infarction in the human is selected from the group consisting of GATA4, Irx4 and Nkx2.5 (also referred to herein as "Nkx2.5/CSX").

The cell populations obtained by the methods of the invention may be administered proximate to the location of the cardiac condition (e.g., myocardial infarction, congestive heart failure). For example, in a human suffering from a myocardial infarction, the cell populations obtained by the methods of the invention may be administered proximate to the myocardial infarction (e.g. into the cardiac muscle at the site of the infarct). The cell populations can be administered directly into the cardiac muscle tissue or spaces of the heart (the ventricular or atrial spaces).

Also described herein is a method of treating a myocardial infarction or a congestive heart failure in a human comprising the steps of culturing a source of a cell population (e.g., adult bone marrow cells) under a low oxygen condition; treating the cultured source of the cell population with a protein kinase C inhibitor (e.g., chelerythrine) and a DNA methylation inhibitor (e.g., 5-azacytidine); and administering the treated cell population to the human.

Also described herein is a method of treating a myocardial infarction or a congestive heart failure in a human, comprising the steps of treating the cell population (e.g., adult bone marrow cells) which co-express CD49c and CD90 with a protein kinase C inhibitor (e.g., chelerythrine) and a DNA methylation inhibitor (e.g., 5-azocytidine). The treated cells are administered to the human. The cell population which is treated with the protein kinase C inhibitor and the DNA methylation inhibitor can further include a cell population which co-expresses telomerase. The method can further include selecting from the treated cells, cells which co-express, CD49c, CD90, at least one cardiac-related transcription factor (e.g., GATA4, Irx4, Nkx2.5) and, optionally, telomerase.

Also described herein is a method of forming a committed progenitor cell-type, comprising the step of combining a substantially homogenous population of cells which co-expresses CD49c and CD90 with a population of cells that includes at least one committed progenitor cell type. The committed progenitor cell-type can be formed *in vitro* or *in vivo.* The formation of the committed progenitor cell-type can be formed *in vivo* by administering the substantially homogenous population of cells which co-express CD49c and CD90 into a human. For example, the method can be used to form a neuronal cell by combining the substantially homogenous cell population with a neuronal cell population (e.g., the central or peripheral nervous system).

The population of cells, which are combined with the cells obtained by the methods of the invention to form a committed progenitor cell-type, may be selected from the group consisting of a population of nerve cells and a population of cardiac muscle cells. The population of cells which co-express CD49c and CD90 may also express telomerase. The population of cells which co-express CD49c and CD90 and, optionally, telomerase, may also express at least one cardiac-related transcription factor. The substantially homogenous population of cells used to form a committed progenitor cell-type may be isolated. The substantially homogenous population of cells which are employed in the method of forming the committed progenitor cells may be a substantially homogenous stem cell population.

The isolated cell population which co-expresses CD49c and CD90 obtained by the methods of the invention has a doubling time less than 30 hours when cultured under a low oxygen condition (e.g., less than about 15% volume oxygen, less than about 10% volume oxygen, less than about 5% volume oxygen).

In still another embodiment, the invention is a method of making an isolated cell population which co-expresses CD49c, and CD90 as defined above, which has a doubling time less than 30 hours when cultured under a low oxygen condition (e.g., less than about 15% volume oxygen, less than about 10% volume oxygen, less than about 5% volume oxygen), wherein the isolated cell population is formed by a method, comprising the step of culturing a cell population source at a seeding density of about 100 cells/cm² under the low oxygen condition.

An isolated cell population obtained by the method of the invention finds application in a pharmaceutical composition comprising an isolated population which co-express CD49c and CD90 (e.g., between about 5 X 10⁵ to 2 X 10⁶ cells). In one embodiment, the pharmaceutical composition has at least about 10⁵ substantially homogeneous cells which co-express CD49c and CD90. In another embodiment, the pharmaceutical composition has at least about 10⁶ substantially homogeneous cells which co-express CD49c and CD90. The cells comprising the pharmaceutical composition can also not express CD34 and/or CD45 and/or can express at least one trophic factor selected from the group consisting of BDNF, NGF, NT-3, IL-6, IL-7, IL-11, SCF, MCP-1, matrix metalloproteinase-9 (MMP-9), Cystatin-C and VEGF.

Also described herein is a pharmaceutical composition comprising a substantially homogeneous cell population which co-express CD49c, CD90 and telomerase.

Also described herein is a pharmaceutical composition comprising a substantially homogeneous cell population which co-express a CD49c, CD90, at least one cardiac-related transcription factor and, optionally, telomerase.

Also described herein is a pharmaceutical composition comprising a substantially homogeneous cell population which co-expresses CD49c, CD90, GATA4, Irx4, Nkx2.5 and, optionally telomerase.

The isolated cell population obtained by the methods of the invention which co-express CD49c and CD 90 can be administered to a human suffering from a neurological condition. A "therapeutically beneficial amount" of the isolated population of cells obtained by the methods of the invention is a quantity sufficient to enhance neuronal function in a subject having a neurological condition (e.g., spinal cord injury) to be clinically relevant.

The cells obtained by the method of the invention can be, for example, transplanted, placed or administered, for example, in the central nervous system (e.g., brain or spinal cord), peripheral nervous system or cardiac tissue (e.g. cardiac muscle). The site of placement in the nervous system or cardiac tissue for the cells is determined based on the particular neurological or cardiac condition (e.g., direct injection into the lesioned spinal cord parenchyma, intra-thecal injection, intracardiac injection, or intravenous injection). For example, cells obtained by the method of the invention can be placed in or near the substantia nigra of patients suffering from Parkinson's disease. Similarly, cells can be placed in or near the spinal cord (e.g., cervical, thoracic, lumbar or sacral) of patients suffering from a spinal cord injury. Likewise, cells can be administered into the cardiac muscle (e.g., ventricular or atrial cardiac muscle) in a patient suffering from a myocardial infarction or other cardiac condition.

The cells obtained by the method of the invention can be placed or transplanted in cavities or spaces of the central nervous system, peripheral nervous system, ventricular space of heart, or atrial space of heart. For example, the cells can be placed in the ventricles of the brain, subarachoid space of the spinal cord, vertebral canal of the spinal cord, ventricles or atria of the heart. One skilled in the art would be able to determine the manner (e.g. needle injection or placement, more invasive surgery) most suitable for placement of the cells depending upon the location of the neurological condition and the medical condition of the patient.

In addition, routes of administration of the cells obtained by the method of the invention, or when such cells are admixed with pharmaceutical carriers, include, for example, intramuscular, intravenous, intraarterial, intraperitoneal, subcutaneous routes, oral, or nasal administration.

The cells obtained by the method of the invention which co-express CD49c and CD90 can be administered alone or as admixtures with conventional excipients, for example, pharmaceutically, or physiologically, acceptable organic, or inorganic carrier substances suitable for enteral or parenteral application which do not deleteriously react with the cells of the invention. Suitable pharmaceutically acceptable carriers include water, salt solutions (such as Ringer's solution), alcohols, oils, gelatins and carbohydrates such as lactose, amylose or starch, fatty acid esters, hydroxymethycellulose, and polyvinyl pyrolidine. Such preparations can be sterilized and, if desired, mixed with auxillary agents such as lubricants, preservatives, stabilizers, wetting agents, emulsifiers, salts for influencing osmotic pressure, buffers, coloring, and/or aromatic substances and the like which do not deleteriously react with the cells.

When parenteral application is needed or desired, particularly suitable admixtures for the cells are injectable, sterile solutions, preferably oily or aqueous solutions, as well as suspensions, emulsions, or implants, including suppositories and soaking in GELFOAM^{®}. In particular, carriers for parenteral administration include aqueous solutions of dextrose, saline, pure water, ethanol, glycerol, propylene glycol, peanut oil, sesame oil and polyoxyethylene-block polymers. Pharmaceutical admixtures suitable for use are well-known to those of skill in the art and are described, for example, in Pharmaceutical Sciences (17th Ed., Mack Pub. Co., Easton, PA) and WO 96/05309.

The cells which co-express CD49c and CD90 obtained by the method of the invention can be used alone or in any combination when administered to a human suffering from a neurological condition. For example, steroids or pharmaceutical synthetic drugs can be co-adminstration with the cells Likewise, treatment of spinal cord injury can include the administration/transplantation of the cells obtained by the method of the invention in a human whose spine has been physically stabilized.

The dosage and frequency (single or multiple doses) of the administration or transplantation of the cells to a human, including the actual number of cells transplanted into the human, can vary depending upon a variety of factors, including the particular condition being treated (e.g., neurological condition, cardiac condition, such as a myocardial infarction, degenerative condition, acute injury), size, age, sex, health, body weight, body mass index, diet of the human, nature and extent of symptoms of the neurological condition (e.g., early onset Parkinson's disease versus advanced Parkinson's disease; spinal cord trauma versus partial or complete severing of the spinal cord), or cardiac condition (e.g., congestive heart failure, myocardial infarction); kind of concurrent treatment (e.g., steroids); complications from the neurological or cardiac conditions; extent of tolerance to the treatment or other health-related problems.

Humans with a neurological or cardiac condition can be treated for days (e.g., 30) with cells obtained by the methods of the invention (e.g., about 10⁶ cells), by a several routes of administration (e.g., intrathecal, intravenous).

It is also envisioned that the methods described herein can be employed to treat neurological conditions in mammals other than human mammals. For example, a non-human mammal in need of veterinary treatment, e.g., companion animals (e.g., dogs, cats), farm animals (e.g., cows, sheep, pigs, horses) and laboratory animals (e.g., rats, mice, guinea pigs).

The present invention is further illustrated by the following examples, which are not intended to be limiting in any way.

### EXEMPLIFICATION

### EXAMPLE 1: Isolation of a adherent as colony forming units of cells or "CFUs" from bone marrow aspirates following red blood cell lysis.

Bone marrow cells were aspirated from the iliac crest of healthy adult human volunteers. The red blood cell component of the aspirate was lysed by mixing the aspirate with an ammonium chloride buffer consisting of 155 mM ammonium chloride, 10 mM potassium bicarbonate and 0.1 mM EDTA (ethylenediaminetetraacetic acid), pH 7.2, at a 1:20 ratio of marrow aspirate to buffer. The resulting cell suspension was vortexed for 2 seconds, incubated for 2 minutes at ambient temperature and then centrifuged (10 minute at 500 x g). The resulting mononuclear cell pellet was resuspended in complete medium and centrifuged (10 minutes at 500 x g). Complete media is Minimal Essential Medium-alpha (Gibco BRL, Rockville, MD) supplemented with 4 mM glutamine and 10% sera-lot selected fetal bovine serum (FBS, Gibco BRL, Rockville, MD). The cell pellet was then re-suspended in the complete medium and centrifuged a second time (10 minutes at 500 x g).

The resulting pellet was re-suspended in the complete medium and the number of viable cells was determined by trypan blue-exclusion. The mononuclear cell suspension was then seeded in tissue culture-treated T75 flask at a density of 50,000 cells/cm2 and incubated at 37°C in an atmosphere consisting of 5% carbon dioxide, 5% oxygen, and 90% nitrogen. On the fifth day of culture, the non-adherent cells and conditioned media (also referred to herein as "spent media") were aspirated from the flasks and the adherent cells re-fed with fresh complete medium. The adherent colony forming units (CFUs) were expanded for an additional 3-5 days.

The generation of CFUs was monitored in 6-well plates concurrently initiated under identical conditions to the T75 flasks. The spent medium was removed from the 6-well plates and the adherent cells were fixed for 5 minutes in 100% methanol, and then stained with methylene blue to visualize the CFUs. An initial seeding density of 75,000 cells/cm² efficiently generated CFUs. After processing the bone marrow aspirate by either FICOLL^{®} density gradient separation or ammonium chloride lysis, CFU efficiency was dramatically affected by oxygen concentration.

After 7 days in culture, the purity (percentage of cells which co-express CD49c and CD90) of the CFUs generated was determined by flow cytometry. T75 flasks were washed twice with Hank's Balanced Salt Solution (HBSS; CellGro Technologies) and treated with 0.1% Trypsin/1 mM EDTA solution (Life Technologies) for 10 minutes at 37°C. Cultures were removed from incubator and 10 mL of complete medium was added. Cells were triturated from the flask, transferred to a 50 mL centrifuge tube and centrifuged (500 x g for 5 minutes). The resulting pellet was resuspended in10 mL of HBSS.

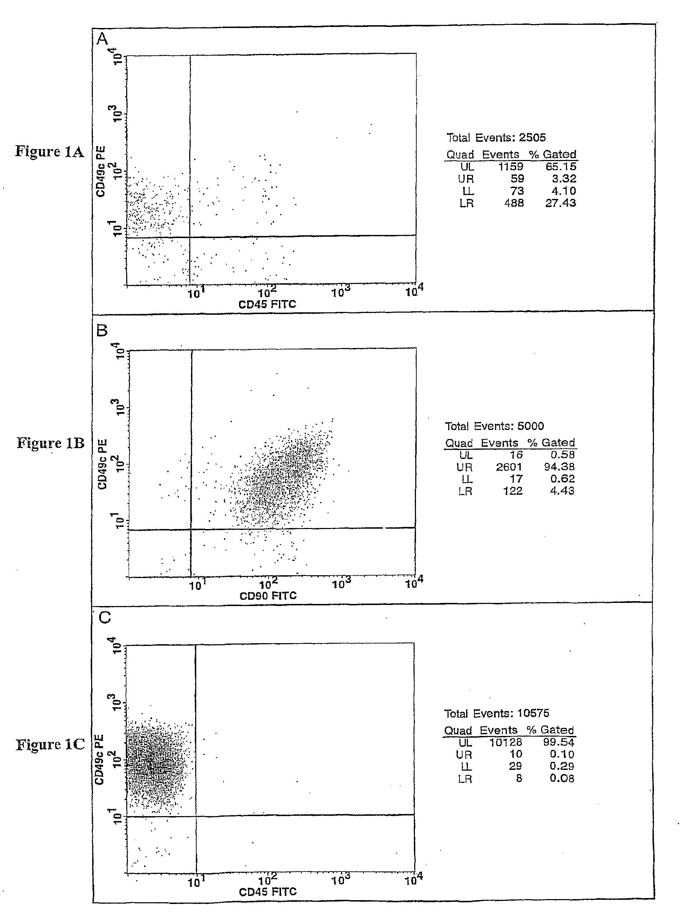

Resuspended cells (approximately 10⁶) were aliquoted into 12x75 mm Flow Cytometry tubes and repelleted at 500 x g for 5 minutes. The HBSS was removed and 25 mL of the following antibodies (all obtained from Becton Dickenson), alone or in combination, were placed into each tube: mouse IgGlk-FITC or -PE (clone MOPC-21) CD49c-PE (cl. C3II.1), CD90-FITC (cl. 5E10), CD45-FITC or -PE (cl. HI30). Tubes were gently vortexed and incubated for 30 minutes at 4°C. Cells were then washed in HBSS/1% bovine serum albumin, centrifuged (30 min, 4°C) and the resulting cellular pellet fixed by the addition of 250 microliters of 2% paraformaldhyde/HBSS. Flow cytometric analysis was performed employing a Becton-Dickenson FACSVantage SE cytometer and analyzed using CELLQUEST^{®} software. Figures 1A, 1B and 1C depict results representing data collected from 2,500-10,000 events per panel. After compensation for non-specific antibody staining using mouse IgGl isotype controls, cellular expression of CD45, CD49c and CD90 in the cultured bone marrow cells was assessed. The adherent population derived from mononuclear cells initially purified using ammonium chloride lysis contained approximately 70% CD49c positive cells at a similar stage of culture (Figure 1A). The majority of cells that did not express CD49c were positive for expression of hematopoetic/myeloid lineage marker CD45 (Figure 1A, LR quadrant), demonstrating that the CD49c positive cell population derived from human bone marrow isolated was not directly related to known hematopoietic precursors. More than 94% of the adherent population was CD90 and CD49c positive (Figure 1B).

### EXAMPLE 2: Isolation of a adherent CFUs from bone marrow aspirates following density separation.

Bone marrow cells were aspirated from the iliac crest of healthy adult human volunteers. The bone marrow aspirate was diluted with calcium and magnesium free phosphate buffered saline (PBS) to achieve a mononuclear cell concentration of 7 x 10⁶ cells/mL and overlaid onto an equal volume of HISTOPAQUE^{®} 1.119 (Sigma, St. Louis, MO) and centrifuged (30 min at 700 x g). The resulting mononuclear cell fraction was transferred to a clean centrifuge tube containing PBS and centrifuged (10 minutes at 500 x g). The cell pellet was re-suspended in PBS and centrifuged (10 minutes at 500 x g). The supernatant was aspirated from the cell pellet and the cells re-suspended in complete media.

The number of viable cells in the resulting cell suspension was determined by trypan blue-exclusion. The cell suspension was then seeded in tissue culture-treated T75 flasks at a density of 50,000 cells/cm² and incubated at 37°C in an atmosphere of 5% carbon dioxide, 5% oxygen and 90% nitrogen. On the fifth day of culture, the non-adherent cells and conditioned media (also referred to herein as "spent media") was aspirated from the flasks and the adherent cells re-fed with fresh complete medium. The adherent CFUs were expanded for an additional 3-5 days.

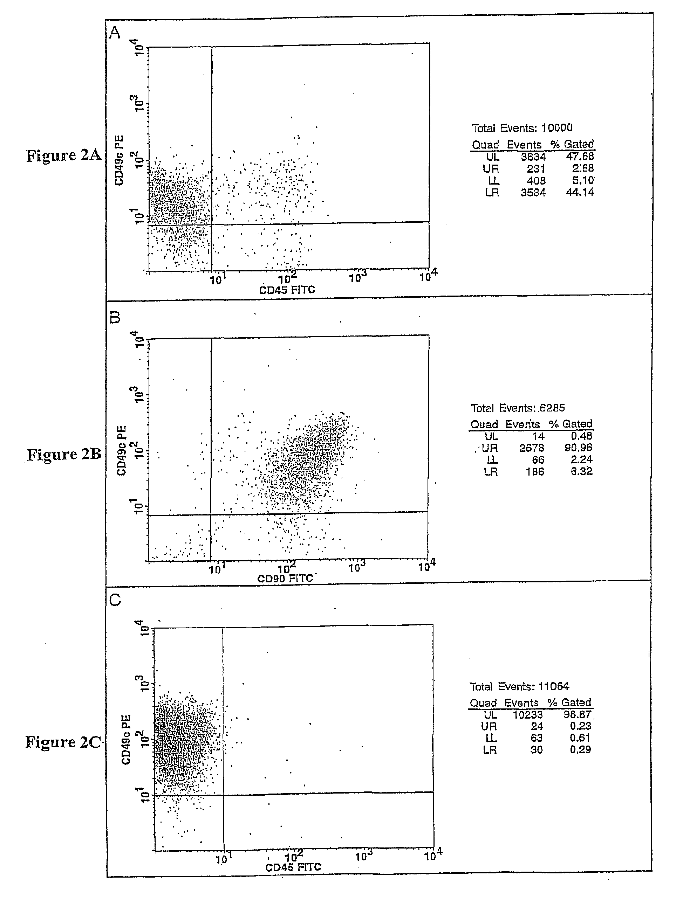

Cytometry analysis of the CFU generated showed that approximately 50% of the adherent population expressed the marker CD49c at 7 days in vitro (Figure 2A, sum of UL and UR quadrants). The majority of cells that did not express CD49c were positive for expression of hematopoetic/myeloid lineage marker CD45 (Figure 2A, LR quadrant), demonstrating that the CD49c positive cell population derived from human bone marrow isolated by this procedure was not directly related to known hematopoietic precursors. More than 91% of the adherent population was CD90 and CD49c positive (Figure 2B).

### EXAMPLE 3: Production of Primary and Master Cell Banks from CFUs.

After 7-10 days in culture, the CFUs generated using the methods described in Example 1 were removed from the T75 flasks with a 0.25% trypsin /1 mM EDTA solution (Life Technologies). After 10 minutes at 37°C, the trypsin was inactivated with 10 mL of complete medium. The cells were washed once with HBSS and re-suspended in Glycerol Cell Freezing Medium^{®} (Sigma Chemical Co.). Aliquots (referred to herein as the "Primary Cell Bank," or "Seed Cell Bank") of the suspension consisting of 4.0 x 10⁵ cells/vial were cooled with liquid nitrogen vapor at 1°C/minute using a CryoMed (Forma) controlled rate freezer and the stored in a Cryo Plus liquid nitrogen storage tank (Forma).

An aliquot of cells was removed from the Primary Cell Bank and cultured at a density of 30 cells/cm² in 500 cm² tissue culture-treated plates (Corning) in complete medium and incubated at 37°C in an atmosphere consisting of 5% carbon dioxide, 5% oxygen, and 90% nitrogen. After two weeks of culture, cells were removed from the plates with trypsin and were cryopreserved at 4.0 X 10⁵ cells/vial (referred to herein as the "Master Cell Bank," or "Production Cell Bank").

The purity of the cells (percentage of cells which co-express CD49c/CD90) in the Master Cell Bank was determined by flow cytometry using the same method as above. The vast majority (>98%) of the resulting population expressed CD90 (Figure 1 C) and virtually lacked any expression of the myeloid-related marker CD45 (Figure 1C, LR quadrant). Thus, the expansion procedure as described herein produces a substantially homogenous population of adherent cells which co-express CD49c and CD90 and lack significant expression of the marker CD45.

Similarly, the master cell bank generated from the CFU derived using method of Example 2 showed that the majority of cells (>98.8%) of the resulting cell population expressed CD90 (Figure 2C) and virtually lacked any expression of the myeloid-related marker CD45 (Figure 2C, LR quadrant). Thus, the expansion procedure as described herein generates a substantially homogenous population of adherent cells which co-express CD49c and CD90 and lack significant expression of the marker CD45.

### EXAMPLE 4: Expansion capability of the cell population which co-express CD49c and CD90

A Primary Cell Bank of CFUs was derived from 25 mLs of bone marrow aspirate and stored as frozen aliquots using the methods of Examples 1 and 2. An aliquot was thawed, expanded and frozen to generate the Master Cell Bank as described in Example 3. An aliquot of cells was removed from the Master Cell Bank and cultured at a density of 30 cells/cm² in 500 cm² tissue culture-treated plates (Corning) in complete medium and incubated at 37°C in an atmosphere consisting of 5% carbon dioxide, 5% oxygen, and 90% nitrogen. After two weeks of culture, cells were removed from the plates with trypsin and cryopreserved at 2-10 x 10⁶ cells/vial. The process was repeated in succession to produce additional Cell Banks of cells which co-express CD49c and CD90.

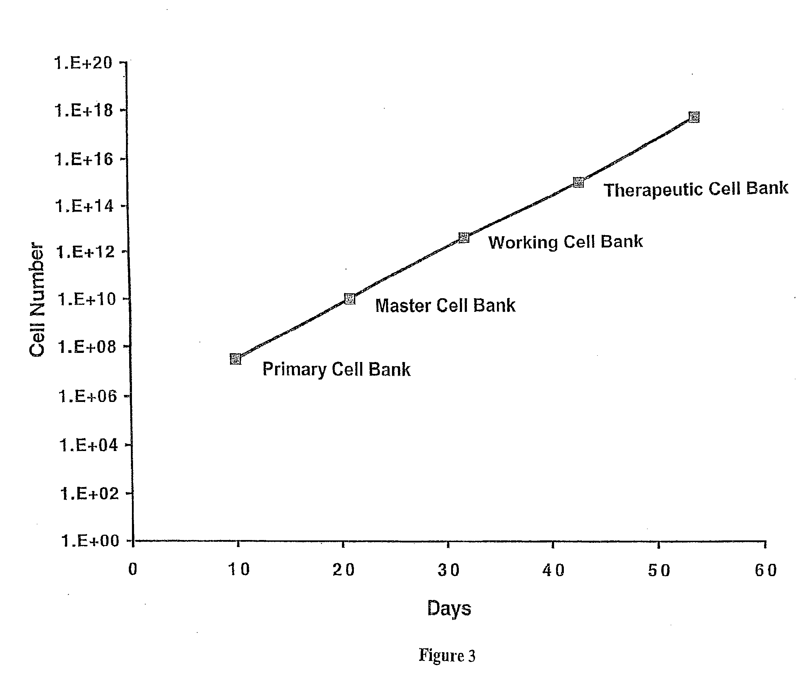

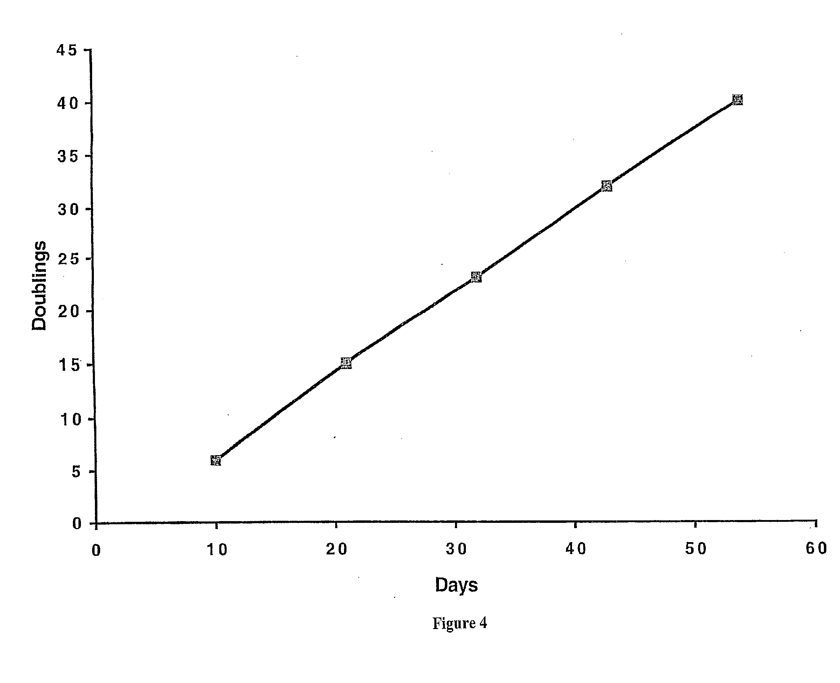

The cell number generated from a single aliquot at the end of each successive expansion was determined by trypan exclusion and multiplied by the number of aliquots to calculate the yield (Figure 3). Four successive expansions can potentially generate up to 1 x 10¹⁷ cells from 25 mLs of bone marrow aspirate obtained from a single donor. The number of cell doublings was calculated from the cell yields using the following formula: (Log(end cell #) - Log (starting cell #)/Log(2) ("(2)" denotes doubling). The cell population underwent about 8 doublings during each expansion (Figure 4). The doubling rate (# days in culture x 24/doublings) was 30 hrs and remained constant for at least 50 doublings. Even after 30 doublings, the population uniformly retained the characteristic morphology of small, dividing cells without apparent evidence of the enlarged, flat morphology of aged or terminally-differentiated cells.

### EXAMPLE 5: Expression of transcripts encoding regulators of cell growth and osteoblast differentiation by cell populations which co-express CD49c and CD90

The expression of transcripts for telomerase, p21, p53, CBFA1 and BSP were determined using quantitative polymerase chain reaction (qPCR). Briefly, Master Cell Bank of CFUs were derived from a bone marrow aspirate and stored as frozen aliquots using the method of Example 1. An aliquot was thawed, cultured at a density of 30 cells/cm² in tissue culture-treated T75 flasks in complete medium and incubated at 37°C in an atmosphere consisting of 5% carbon dioxide, 5% oxygen, and 90% nitrogen.

After two weeks of culture, the cells were seeded in 96 well plates at 3000 cells/well. RNA was isolated using the QIAGEN RNeasy reagents and the Qiagen Biorobot 3000. An aliquot of the eluted RNA was used to synthesize cDNA. RNA was mixed with Promega MMLV, dNTPS, decamers and RNasin and incubated at 37°C for 1 hour, followed by heat inactivation. For quantitative PCR (qPCR), cDNA samples were combined with Applied Biosystems SYBR Green PCR Core reagents and amplicon specific primers, as described below, in a 384 well format. The 384 well plate was then transferred to the Applied Biosystems ABI Prism 7900 for qPCR analysis. The qPCR program entailed a 2 minute cycle at 50°C, followed by a 10 minute cycle at 95°C to activate the polymerase. This was than followed by 40 amplification cycles consisting of 15 seconds of melting at 95°C and one minute of extension/annealing at 60°C.

Cycle threshold values were converted into relative transcript number using a standard curve then normalized using the corresponding 18s. Data are expressed as a ratio of transcript per 10⁶ 18s transcripts. The name, Genebank ID, bp location and sequence of the qPCR primers are as follows:
18s-1F, K03432, 1742-1760bp, 5'-ATG GGG ATC GGG GAT TGC A-3' (SEQ ID NO: 1);
18s-1R, K03432, 1871-1890bp, 5'-CCG ATC CGA GGG CCT CAC TA-3' (SEQ ID NO: 2);
BSP-1F, NM000582, 483-508bp, 5'-CAC TCC AGT TGT CCC CAC AGT AGA CA3' (SEQ ID NO: 3);
BSP-1R, 611-632bp, 5'-TCG CTT TCC ATG TGT GAG GTG A-3' (SEQ ID NO: 4);
CBFA1-1F, L40992, 389-407bp, 5'-GGC CGG AGT GGA CGA GGC AA-3' (SEQ ID NO: 5);
CBFA1-1R, L40992, 504-529bp, 5'-CAT CAA GCT TCT GTC TGT GCC TTC TG-3' (SEQ ID NO: 6);
p21-1F, 567388, 52-72bp, 5'- ACC GAG GCA CTC AGA GGA GGC-3' (SEQ ID NO: 7);
p21-1R, S67388,171-191bp, 5'- GCC ATT AGC GCA TCA CAG TCG-3' (SEQ ID NO: 8);
p53-qFP4, M14694, 521-545bp, 5'- GAT GTT TTG CCA ACT GGC CAA GAC C-3' (SEQ ID NO: 9);
p53-qRP4, M14694, 674-698bp, 5'- AGG AGG GGC CAG ACC ATC GCT ATC T-3' (SEQ ID NO: 10);
Telo-1F; AF015950, 1500-1525bp, 5'-ACA ACG AAC GCC GCT TCC TCA GGA AC-3' (SEQ ID NO: 11); and
Telo-1R, AF015950, 1625-1650bp, 5'-GCC GGA ACA CAG CCA ACC CCT GG-3' (SEQ ID NO: 12).

Telomerase activity is necessary for maintaining telomeres, which are DNA sequences located at the ends of chromosomes. Since most human cells lack telomerase, the telomeres shorten with each division until the cells growth arrest (Harley, C.B., Mutation Research 256(2-6):271-282 (1991); Hara, E. et al., Biochem Biophys Res Commun 179(1):528-534 (1991); Shay, J.W. et al., Exp Cell Res 196(1):33-39 (1991)). Cell populations which co-express CD49c and CD90 express telomerase at the level of approximately 13 transcripts/10⁶ transcripts of 18S rRNA, which is consistent with the finding that this cell population continued to proliferate at a constant rate.

The p53 tumor suppressor plays a key role in the cell's response to DNA damage and inactivation of this gene is an important step in carcinogenesis. p53 expression is upregulated in response to DNA damage. Its ability to prevent the proliferation of defective cells involves the activation of several growth arrest genes including p21 (Bums, T.F. et al., Oncogene 20(34):4601-4612 (2001)). The substantially homogenous cell population of the invention which co-express CD49c and CD90 expressed about 670 p53 transcripts/10⁶ transcripts of 18S rRNA. This level of p53 shows that the tumor suppressor p53 was not induced.

p21 is a potent cell cycle inhibitor and its expression during the cell cycle is tightly regulated at the transcriptional level (Gartel, A.L. et al., Exp Cell Res 246(2):280-289 (1999)). p21 is induced in growth arrested cells in response to oxidative stress in addition to DNA damage (Yin, Y., et al., Mol Carcinog 24(1):15-24 (1999)). The substantially homogenous cell population of the invention which co-express CD49c and CD90 expressed p21 about 1690 transcripts/10⁶ transcripts of 18s rRNA. This level of p21 shows that p21 was not induced and is consistent with both a low level of p53 and the short doubling time measured in Example 4.

The transcription factor, CBFA1, is necessary for osteoblast differentiation and bone formation (Otto, F., Cell 89(5):765-771 (1997)). Bone sialoprotein (BSP) is a prominent, mineral-associated protein in the extracellular matrix of bone and is expressed by fully differentiated osteoblasts (Benson, M.D., et al., J Biol Chem 275(18):13907-13917 (2000)). The substantially homogenous cell population of the invention which co-express CD49c and CD90 expressed about 130 CBFA1 transcripts/10⁶ transcripts of 18S rRNA and BSP was absent, which show that the cell population of the invention represents a progenitor that has not significantly differentiated into osteoblasts. Osteoblast differentiation is described in Ducy, P., Dev Dyn 219(4):461-471 (2000)).

### EXAMPLE 6: Secretion of neurotrophic factors and cytokines by a substantially homogenous cell population which co-express CD49c and CD90.

An aliquot of the Master Cell Bank generated in Example 1 was thawed and plated onto T75 flasks at 2500 cells/cm² with complete medium and incubated at 5%O₂. The following day the medium was removed and replaced with fresh complete medium. The supernatant was collected 8 hours later from T75 and the cells were counted (Cell count = 280,000 cells). The supernatant was aliquoted to 1 ml tubes and stored at -20°C. Another T75 was processed the same way 3 days later (Cell count = 2.43 million). Supernatants were later thawed at room temperature and assayed by ELISA for secretion of the following neurotrophicfactors/cytokines using commercially available kits: BDNF (Chemicon), NGF (Chemicon), MCP-1 (R and D Systems), and IL-6 (R and D Systems). Multiple dilutions were performed on supernatant to ensure that measured values fell within standard ranges of the assay. In addition, media obtained from control cells secreting previously determined amounts of cytokine were run in parallel to assure assay validity. Values were obtained by normalizing raw data derived from ELISA to standard time (24 hours) and cell number (1 million) and are thus expressed as "picograms of cytokine secreted per 1 million cells per 24 hour period as follows:

| **Cytokine** | **Amount Secreted (pg/10⁶ cells/day** |
|---|---|
| MCP-1 | 1009.15 |
| IL-6 | 18567.60 |
| BDNF | 8.88 |
| NGF | 80.12 |

### EXAMPLE 7: Transplantation of a substantially homogenous cell population of the invention which co-express CD49c and CD90 into an acute rat spinal cord injury model

Following traumatic injury to the spinal cord neuronal death, inflammation and progressive loss of damaged neurons ensue overtime.. A substantially homogenous cell population of the invention which co-express CD49c and CD90 improved outcome during acute neurologic injury. A cell population prepared as described in Example 1 was transplanted into contused spinal cord of adult female Sprague-Dawley rats.

The thoracic spinal cord of Sprague-Dawley rats was exposed by laminectomy at the level of T10 under general anesthesia. After the laminectomy is completed, a 10 gm rod was dropped from a height of 25 mm to produce a spinal cord injury of moderate severity onto the exposed spinal cord using the NYU spinal cord impactor (Constantini, S. et al., J Neurosurg 80(1):97-111 (1994)). During surgery, the body temperature of the rats was kept at 37°C. During recovery, rats were placed overnight in a temperature and humidity controlled chamber. Seven days after impact injury, the spinal cords were re-exposed. Using a 50 mL gas tight Hamilton syringe (VWR Scientific Products, Bridgeport, NJ) with a 30 gauge needle, 250,000 cells of the invention at a concentration of 25,000/µl were transplanted into the spinal cord. The cells were injected into the epicenter of the syrninx at the T10 level at a rate of 2 mL/minute. The needle was left in place for additional 5 minutes before it was removed from the spinal cord. Following surgery, all animals received 30mg/kg methylprednisolone intravenously (i.v.) immediately following surgery. To prevent immun-rejection, Cyclosporin A (CsA) was given subcutaneously at 10mg/kg 3 days prior to the day of transplantation and maintained thereafter.

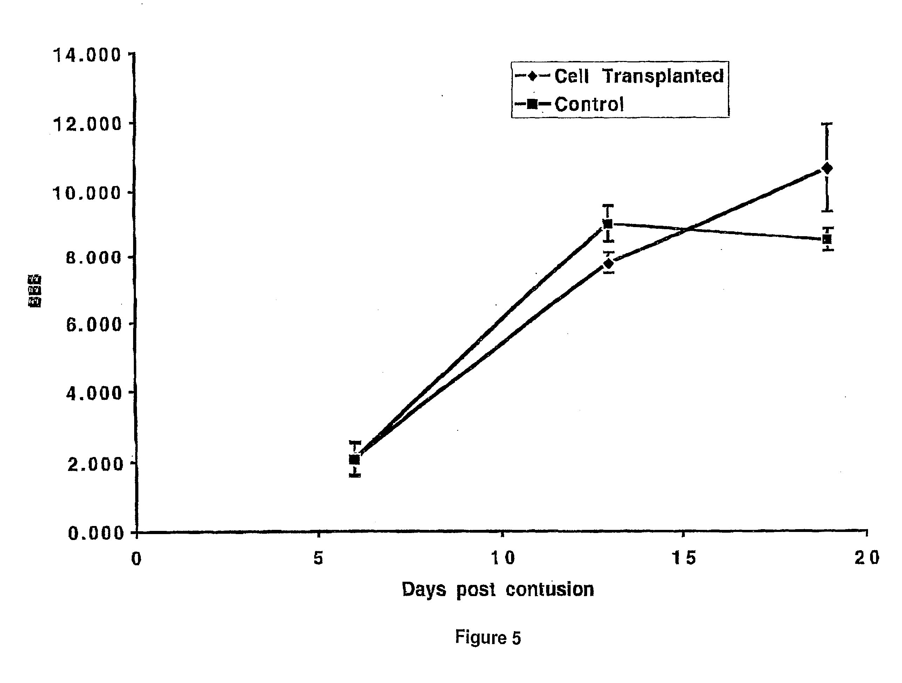

The Basso-Beattie-Bresnahan openfield locomotor test (BBB Test) (Basso, D.B. et al., J Neurotrauma 12(1):1-21 (1995)) were performed the day before transplantation (day 6 after injury). Behavioral testing was performed for each hindlimb weekly using the BBB scores. Scoring was performed blinded to the treatment status. At 19 days after contusion, animals that had received a substantially homogenous population of cells which co-express CD49c and CD90, showed greater improvement on the BBB score than animals received only PBS (10.6 ± 1.2 vs 8.5 ± 0.3) (Figure 5).

At 2 weeks after transplantation, contused spinal cords from some animals were removed and examined for evidence on nerve fiber regeneration. SMI 32 (Sternberger Monoclonals, Inc, Lutherville, MD) antibody was used to immunostain for regenerating fibers in the syrinx at a dilution of 1:4000. Numerous fibers were observed growing into the contused syrinx.

### Example 8: Expression of transcripts encoding regulators of neuronal differentiation by a substantially homogenous population of cells which co-express CD49c and CD90.

The expression of transcripts for Sox-2 and Musashi were determined using quantitative polymerase chain reaction (qPCR). Briefly, Master Cell Bank of CFUs were derived from a bone marrow aspirate and stored as frozen aliquots using the method of Example 1. An aliquot was thawed and the cells were seeded in 96 well plates at 2000 cells/well in complete media and incubated at 37°C in an atmosphere consisting of 5% carbon dioxide, 5% oxygen and 90% nitrogen for 3 days. On the third day of culture, cells were treated with 5 µM nifedipine (an L-type calcium channel blocker). After 24 hours of treatment, RNA was isolated using the QIAGEN®RNeasy reagents and the Qiagen Biorobot 3000. An aliquot of the eluted RNA was used to synthesize cDNA. Specifically, RNA was mixed with Promega MMLV, dNTPS, decamers and RNasin and incubated at 37°C for 1 hour, followed by heat inactivation.

For quantitative PCR, cDNA samples were combined with Applied Biosystems SYBR Green PCR Core reagents and amplicon specific primers, as described below, in a 384 well format. The 384 well plate was then transferred to the Applied Biosystems ABI Prism 7900 for qPCR analysis. The qCPR program entailed a 2 minute cycle at 50 degrees, followed by a 10 minute cycle at 95 degrees to activate the polymerase. This was then followed by 40 amplification cycles consisting of 15 seconds of melting at 95 degrees and one minute of extension/annealing at 60 degrees. Cycle threshold values were converted into relative transcript number using a standard curve then normalized using the corresponding 18s. Data are expressed as a ratio of transcript per 10⁶ 18s transcripts.

The name, Genebank ID, bp location and sequence of the qPCR primers are as follows:
18s-1F, K03432,1742-1760bp, 5'-ATG GGG ATC GGG GAT TGC A-3' (SEQ ID NO: 1);
18s-1R, K03432,1871-1890bp, 5'-CCG ATC CGA GGG CCT CAC TA-3' (SEQ ID NO:2);
Sox-2F, Z31560, 517-541bp, 5'-GGC AGC TAC AGC ATG ATG CAG GAC C-3' (SEQ ID NO: 13);
Sox-2R, 624-647 bp, 5'-CTG GTC ATG GAG TTG TAC TGC AGG-3' (SEQ ID NO: 14);
Musashi-1F, AB012851, 370-389 bp, 5'-CAA GAT GGT GAC TCG AAC GA-3' (SEQ ID NO:15);
Musashi-1R, 480-499 bp, 5'-GGT TTT GTC AAA CAT CAG CA-3' (SEQ ID NO: 16).

The gene Sox-2 encodes a conserved nuclear transcription factor related to the Mammalian Testis Determining Gene that is expressed throughout the neural tube during brain development and is essential for the survival of primitive neural ectoderm (Uwanogho, Mech. Dev. 49:23-36 (1995)). In addition, expression of Sox-2 persists beyond development in restricted populations of adult neural stem cells, suggesting a further role for this factor in regulating neural fate (Zappone, Development 127:2367-2382 (2000)). Under unstimulated conditions in complete media, the cells which co-expressed CD49c and CD90 expressed approximately 4 Sox-2 transcripts/10⁶ transcripts of 18S RNA. However, in response to nifedipine, the cells which co-expressed CD49c and CD90 expressed 28 Sox-2 transcripts/10⁶ transcripts of 18S RNA, an approximate 7-fold increase. This increase in Sox-2 expression suggests that, in response to certain epigenetic treatments, the cells which co-expressed CD49c and CD90 can display traits associated with early neural populations.

The gene Musashi encodes an RNA-binding protein that is highly expressed within the developing nervous system and, like Sox-2, is also expressed in mammalian neural stem cells (Sakakibara, Dev Biol 176:230-242 (1996)). Furthermore, expression of Musashi is required for normal development of multiple neuronal population (Nakamura, Neuron 13:67-81 (1994)). Under unstimulated conditions in complete media, the cells which co-expressed CD49c and CD90 expressed approximately .005 Musashi transcripts/10⁶ transcripts of 18S RNA. However, in response to 24 hours of stimulation with nifedipine, the cells which co-expressed CD49c and CD90 expressed .093 transcripts/10⁶ transcripts of 18S RNA, an approximate 17-fold increase. This increase in Sox-2 expression suggests that, in response to certain epigenetic treatments, the cells which co-expressed CD49c and CD90 can display traits associated with early neural populations.

### EXAMPLE 9: The Effect of Intra-Cardiac Injection of Human Adult Bone Marrow Stem Cells (hABM-SC) on Myocardial Infarction

The ability of hABM-SCs in restoring cardiac function after direct intra-cardiac injection in a rat animal model induced with experimental myocardial infarct was determined. The distribution and disposition of the hABM-SCs in these animals can be determined.

### Materials and Methods

### EarIy-Myocytic Determined Cells (EMD) for Intra-Cardiac Injection

hABM-SC were cultured under conditions which induce the expression of cardiac-related transcription factors to produce early-myocytic determined cells. Early-myocytic determined cells were obtained by culturing hABM-SCs in the presence of a DNA methylation inhibitor (e.g., azacytidine) and a protein kinase C inhibitor (e.g., chelerythrine). The early-myocytic determined cells of the invention co-express CD49c, CD90 and at least one cardiac-related transcription factor such as Nkx2.5, Irx4 and GATA4.

"Early-myocytic determined cells ("EMD")," as used herein, refers to cells that are partially differentiated into cardiomyocytes. Criteria to determine whether a cell is an early-myocytic determined cell include, for example, expression of at least one cardiac-related transcription factor by the cell. The early-myocytic determined cells can become cardiac cells (e.g., cardiac muscle cells, also referred to herein as cardiomyocytes).

Cardiomyogenesis, the development or differentiation of cardiomyocytes, is characterized by a time-specific and distribution-specific expression of various proteins and genes. The gene Nkx2.5/CSX (cardio specific homeobox) has been identified as one of the earliest genes expressed during heart development, followed closely by another homeobox gene, the Iroquois homeobox gene4 (Irx4) (Bao, Z., et al., Science 283: 1161-1164 (1999); Auda-Boucher, G., et al., Dev. Biol. 225: 214-225 (2000)). The transcriptional regulator, GATA-binding transcription factor 4 (GATA4), is abundant in the heart and important in myocyte differentiation (Auda-Boucher, G., et al., Dev. Biol. 225:214-225 (2000)). Co-expression of Nkx2.5, Irx4 and GATA4 is unique to cardiac muscle cells (Mably, J.D., et al., Circulation Rec. 79(1):4-13 (1996)). Co-expression of transcripts for Nkx2.5, Irx4 and GATA4 in hABM-SC was achieved by culturing hABM-SCs under the following conditions.

Master Cell Bank cells were cultured in a 96 well format in complete medium and incubated at 37°C in an atmosphere consisting of 5% carbon dioxide, 5% oxygen and 90% nitrogen. After 2 days in culture, cells were cultured in the presence of 30µM 5-azacytidine (a DNA methylation inhibitor) and 5µM chelerythrine (a protein kinase C inhibitor) for 3-7 days in complete media. Other protein kinase C inhibitors, such as H-7 dihydrochloride, K252a, staurosporine, bisindolylmaleimide I-V, and calphostin C, can also employed in the methods and culture conditions of the invention to produce early-myocytic differentiated cells. In addition, other DNA methylation inhibitors, such as 5-aza-2-deoxycytide, 5-azaguanine, 5-aza-2-deoxyguanine, can also be used in the methods and culture conditions of the invention to produce early-myocytic differentiated cells.

After 2 days of culturing in the presence of azacytidine and chelerythrine, RNA was isolated from the cells in culture. An aliquot of the eluted RNA was used to synthesize cDNAs for analysis of the expression of cardiac-related transcription factors. RNA was mixed with Promega Moloney Murine Leukemia Virus (MMLV) reverse transcriptase. cDNAs, dNPTs, decamers and RNasin were incubated for about 1 hour at about 37°C, followed by heat inactivation. For quantitative polymerase chain reaction (qPCR), cDNA samples were combined with Applied Biosystems SYBRR GREEN PCR 2X MASTER MIX^{®} and amplicon specific primers in a 384 well format. The ABI PRISM 7900 HT^{®} performed the qPCR using a standard program of about 2 minutes at about 50°C, about 10 minutes at about 95°C, about 40 amplification cycles consisting of about 15 seconds of melting at about 95°C and one minute of annealing/extension at about 60°C. Cycle threshold values were converted into relative transcript number using a standard curve and then normalized to the corresponding 18s RNA transcripts. Data were expressed as a ratio of transcript per 10⁶ 18s RNA transcripts. Control values for transcripts evaluated were in the range of 0.001-0.003. GATA4 was induced to 0.71 transcripts, Irx4 to a level of 0.023 transcripts and Nkx2.5 to 0.023 transcripts, all relative to 10⁶ 18s RNA. Cells which expressed at least one cardiac-related transcription factor, e.g., GATA4, Irx4 and Nkx2.5, are early-myocytic determined cells or early-myocytic determined hABM-SCs.

Early-myocytic determined cells, which co-express GATA4, Irx4 and Nkx2.5, were plated at about 30 cells/cm² with complete media and incubated at about 37°C in an atmosphere consisting of about 5% carbon dioxide, about 5% oxygen and about 90% nitrogen. After 7 days in culture all media was replaced with new complete media containing 30µM 5-azacytidine and 5µM chelerythrine and cells cultured for about 3 days followed by a complete media change and cultured an additional 7 days. Early-myocytic determined cells had a doubling time about twice that of unmodified cells. The early-myocytic determined cells can secrete trophic factors such as IL-6, VEGF, MCPI and BDNF.

### Unmodified Cells for Intra-Cardiac Injections

Donor 059-derived hABM-SCs, which were not cultured in the presence of a DNA methylation inhibitor (e.g., 5-azacytidine) and a protein kinase C inhibitor (e.g., chelerythrine chloride) are referred to herein in this example as "unmodified hABM-SCs" or "unmodified cells." Unmodified hABM-SCs were a substantially homogenous cell population which co-expressed CD49c, CD90 and telomerase; and were prepared as described in Examples 1-8.

### Experimental Induction of Myocardial Infarction

Myocardial infarction was experimentally induced in male and female Sprague-Dawley rats (age about 3 months) by placement of a permanent silk ligature around the left-anterior descending (LAD) coronary artery through a midline sternotomy according to established methods (Müller-Ehmsen, J. et al., Circulation 105:1720-1726 (2002)). Five days after the procedure, rats were treated with a standard regimen of 10mg/kg Cyclosporine A treatment that lasted for the duration of the 4 week study.

About seven to eight days after induction of the infarction, rats were anesthetized, intubated and an intercostal incision was made to expose the apex of the heart. An ultrasonic Millar catheter was inserted through the ventricular wall, and pressure over time measurements, dp/dt, obtained for about 30-60 seconds. This model of infarct production and pressure/time measurements of cardiac function is a standard, well characterized model by which the effects of cellular therapies on cardiac function are assessed (Müller-Ehmsen, J. et al., Circulation 105:1720-1726 (2002)). After baseline measurements, rats were placed in one of three treatment groups outlined in Table 1.

**TABLE 1**

| **Group** | **Treatment** | **No. Animals** | **Duration of Treatment** |
|---|---|---|---|
| Vehicle | 100 µL | 7 Males | 4 weeks |
| | | 7 Females | |
| | | | |
| Unmodified | 5 X 10⁶ hABM-SCs/100 µL | 7 Males | 4 weeks |
| Cells | | 7 Females | |
| | | | |
| Early Myocytic | 5 X 10⁶ hABM-SCs/100 µL | 7 Males | 4 weeks |
| Determined Cells | | 7 Females | |

The early-myocytic determined cells, unmodified cells or vehicle (4.5% glucose in PBS) was delivered to the heart using a 100 µL Hamilton syringe fitted with a 30 gauge, low dead-space needle. The dose of early-myocytic determined cells or unmodified cells was five (5) unjections of 20µl of PBS/4.5% glucose containing 1 x 10⁶ cells (total 5 x 10⁶ cells in 100 µl PBS/4.5% glucose). Five separate injections of 20 µL were performed over the course of 2-3 minutes. Four injections were performed at equal distances around the visualized infarct, while the fifth was placed directly into the center of the region with the infarction as determined by area of discoloration indicative of the infarction. After injection, the incision was sutured shut, the pneumothorax was reduced, and the animals were weaned from the respirator and extubated. The rats were returned to their cages upon gaining consciousness. Daily clinical observations were noted for the remaining duration of the study.

Four weeks after injection (5 weeks post-infarction), animals were re-anesthetized, the heart was exposed through a midline sternotomy, and a Millar catheter was inserted. Dp/dt measurements were taken as described above, after which the rats were euthanized via exsanguination. The hearts were harvested, immersion fixed, paraffin embedded, sectioned, stained with hemotoxylin/eosin (H&E) and Trichrome and analyzed histologically with the aid of a light microscope.

Data were blind analyzed by a third party.

Data are expressed as a mean ± standard error of the mean (SEM). Statistically significant differences in mean values was determined by Analysis of Variance (ANOVA).

### Results

Treatment with either modified cells or unmodified cells resulted in significant improvements in several metrics of cardiac function 4 weeks after treatment when compared to vehicle treated control infracted rats. Histological analysis of cardiac tissue supports the functional data.

### Functional Measurements of Cardiac Tissue:

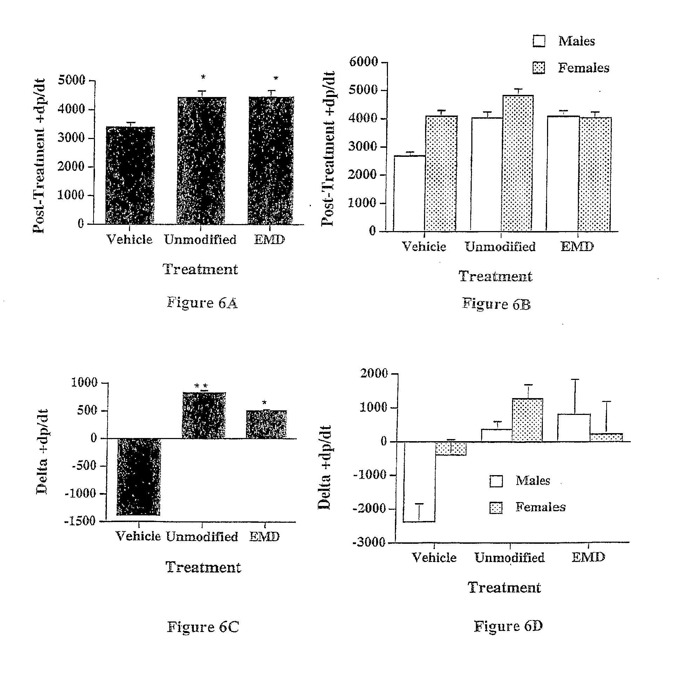

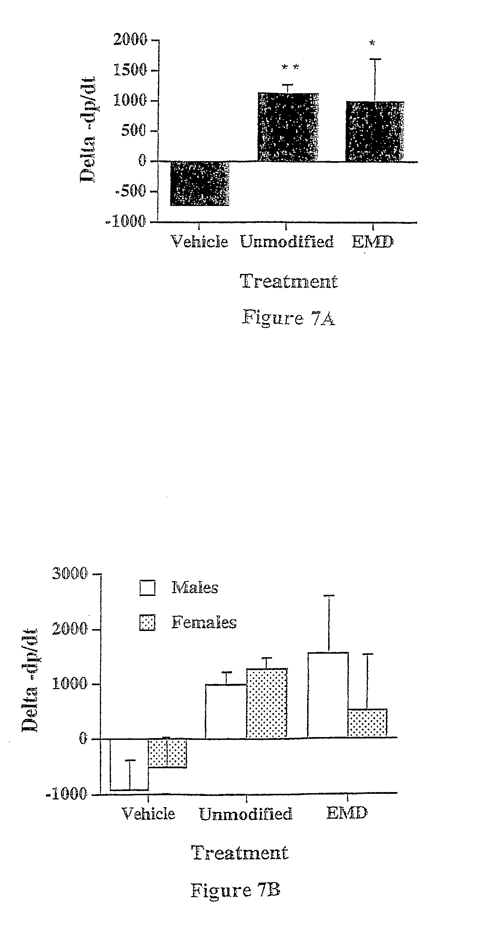

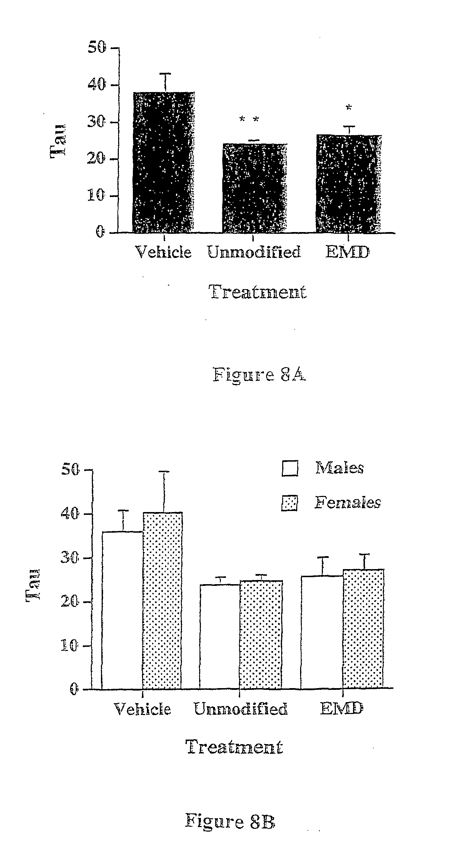

Statistical analyses was performed on functional data. Due to deaths of some rats at treatment time, as well as the loss of two post-treatment data files, functional data for 6 animals/sex/treatment group was generated. Functional data was analyzed as the following metrics: +dp/dt (Figures 6A and 6B), delta +dp/dt (Figures 6C and 6D), delta -dp/dt (Figures 7A and 7B) and as tau (Figures 8A and 8B).

As shown in Figure 6A, treatment with unmodified cells and modified cells resulted in a significant increase in the maximum rate of pressure development (+dp/dt) 4 weeks after injection. Pretreatment values were not significantly different between groups.

As shown in Figure 6B, vehicle treated males had a significantly lower +dp/dt value when compared to females. However, the degree of increase relative to vehicle was about equal in males and females and less in rats treated with modified cells.

As a final confirmation that improvements in +dp/dt measurements were of significance, a metric referred to as "delta +dp/dt" was derived. Delta +dp/dt is the absolute change between pretreatment and post-treatment +dp/dt values taken on each individual animal and averaged by the value of the treatment group (Figure 6C). Delta +dp/dt measurements show that while vehicle animals demonstrated a decrease in this metric (negative delta which indicates decreased cardiac function) during the course of the study, animals receiving either modified cells or unmodified cells demonstrated a significant increase in cardiac function (positive delta which indicates increased cardiac function) as defined by pressure generation metrics.

As shown in Figure 6D, males treated with vehicle had a greater decrease in cardiac function (negative delta) when compared to females treated with vehicle. However, males and females demonstrated equivalent increases (positive delta) after treatment with modified cells or unmodified cells.

The minimum rate of pressure development ("-dp/dt") was also determined. A similar analyses as described for +dp/dt was performed (Figures 7A and 7B). Results are expressed as "delta -dp/dt," although a similar pattern for pre- and post-treatment measurements was observed. Four weeks after treatment, rats receiving either unmodified cells or modified cells demonstrated significantly higher -dp/dt values (not shown). Unlike +dp/dt values, no differences in -dp/dt were observed between male and female rats.

As shown in Figure 7A, the delta -dp/dt decreased in vehicle treated animals. However, animals receiving either modified cells or unmodified cells demonstrated significant and robust increases in cardiac function as defined by delta -dp/dt. Figure 7A shows that expressing changes in cardiac function over the course of the study by subtracting 0 week +dp/dt values from 4 week +dp/dt values ("delta -dp/dt") demonstrated that while vehicle-treated rats had decreases in cardiac function over the course of the study (negative delta), animals treated with either modified cells or unmodified cells showed significant improvements in cardiac function. Unlike +dp/dt, there were no significant differences in the response between males and females (Figure 7B).

A third metric obtained in this study was the time constant of isovolumetric left ventricular pressure decay, termed tau (t) (Figures 8A and 8B). Elevated tau values have been reported in a number of cardiac pathologies in man, including coronary artery disease, myocardial infarct, and diastolic heart failure and, thus, are an index of cardiac function (Bolognesi, R., et al., J. Am. Soc. Echocardiogr. 14(8):764-72 (2001); Dawso, J.R., et al., Br. Heart J. 61(3):248-257 (1989)). Consistent with the +dp/dt and -dp/dt measurement, rats receiving either modified cells or unmodified cells demonstrated a decreased tau value 4 weeks after treatment (Figure 8A), suggesting increased left ventricular compliance. As observed with +dp/dt, vehicle treated males tended toward greater decreases in tau over the course of the study, but both sexes demonstrated equal degrees of recovery in tau in response to both test articles when normalized to respective vehicle control (Figure 8B).

### Histological Analysis Cardiac Tissue:

Sections of hearts were stained with H&E and Trichrome. Trichrome staining allows for the visualization of collagen versus muscle tissue. Since collagen can indicate the presence of scar tissue and, thus, the absence of regeneration, H&E and Trichrome staining can be useful to quantify the relative amounts of collagen in cardiac tissue obtained from animals in the experimental treatment groups (vehicle, unmodified cells, early myocytic determined cells) compared to normal cardiac muscle. The scale depicted in Table 2 was employed to correlate quantitative functional data with histological analysis.

**TABLE 2**

| **Scale** | **Fibrotic/Viable Tissue Area (AF:AV)** |
|---|---|
| 0 | Very little |
| 1 | Little |
| 2 | Mild |
| 3 | Moderate |
| 4 | Large |
| 5 | Very Large |

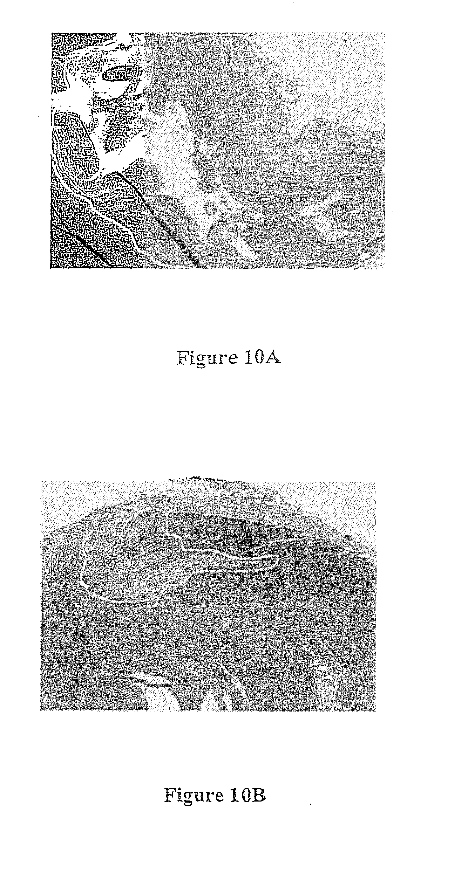

Figures 10A and 10B illustrate the relative differences between a vehicle treated rat (Figure 10A, Score 4) and a rat treated with unmodified cells (Figure 10B, Score 1). The scores were obtained following visual inspection of three slides of cardiac tissue, starting from the cardiac apex, with each section about 4mm apart. The score was rendered based upon an average across the three regions of the heart.

A score of 0 was characterized by little to no fibrosis from the apex of the heart to the atrium in cardiac tissue. A score of 1 was characterized by little fibrosis at the apex of the heart and small amounts of fibrous towards to the atrium. A score of 2 was characterized by mild fibrosis at the apex of the heart and quick decreases in the ratio of fibrotic:viable tissue towards the atrium in cardiac tissue. A score of 3 was characterized by moderate fibrosis at the apex of the heart and quick decreases in the ratio of fibrotic:viable tissue towards the atrium in cardiac tissue. A score of 4 was characterized by large fibrosis at the apex of the heart and less rapid decreases in the ratio of mild to fibrotic: viable tissue towards the atrium in cardiac tissue. A score of 5 was characterized by very large fibrosis at the apex of the heart and linear decreases in the ration of fibrotic:viable tissue towards the atrium in cardiac tissue.

The mean score obtained from rats treated with vehicle alone was (4.4 ± 0.68). The mean score obtained from rats treated with modified (1.33 ± 0.68) or unmodified cells (1.00 ± 0.45). For visual representation of a score of 4 and 1, refer to Figure 10A and 10B, respectively.

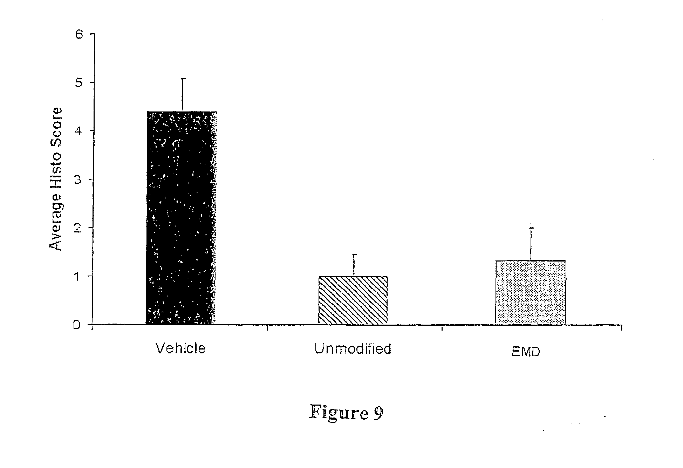

These data show that a significant reduction in infarct size was observed in rats treated with early-myocytic determined cells and unmodified cells. Generally, infarct size in females was smaller than in males. Rats treated with early-myocytic determined cells or unmodified cells had histological scores approximately two points lower than matched gender vehicle controls (Figures 9, 10A and 10B).

## Claims

1. A method of making an isolated cell population derived from human bone marrow, wherein between 100% to 90% of the cells of the cell population co-express CD49c and CD90, and wherein the cell population has a doubling time of less than 30 hours, comprising the steps of:
a) culturing a source of the cell population under a low oxygen condition of less than 15% oxygen to produce an adherent cell population; and,
b) culturing the adherent cell population at a seeding density of less than 2500 cells/cm².

2. The method of claim 1, wherein the source of the cell population in claim 1, part a) is cultured at a seeding density of less than 75000 cells/cm² and the adherent cell population in claim 1, part b) is cultured at a seeding density of less than 100 cells/cm².

3. The method of any one of claims 1 to 2, wherein the adherent cell population in claim 1, part b) is cultured under a dissolved oxygen concentration of less than 20% at a seeding density selected from the group consisting of:
a) less than 1000 cells/cm²;
b) less than 100 cells/cm²;
c) less than 50 cells/cm²; and,
d) less than 30 cells/cm².

4. The method of any one of claims 1 to 3, wherein the low oxygen condition is selected from the group consisting of:
a) between 1 to 10% oxygen;
b) between 2 to 7% oxygen;
c) less than 10% oxygen;
d) less than 5% oxygen; and,
e) 5% oxygen.

5. The method of any one of claims 1, and 2 to 4, further including lysing the source of the cell population prior to culturing the source of the cell population.

6. The method of any one of claims 1 to 4, further including selecting a fractionated source of the cell population by passage through a density gradient prior to culturing the source of the cell population.

7. The method of any one of claims 1 to 6, wherein the cells of the cell population that co-express CD49c and CD90, do not express CD34 and/or CD45.

8. The method of any one of claims 1 to 7, wherein the cells of the cell population that co-express CD49c and CD90 further express at least one cardiac-related transcription factor selected from the group consisting of GATA-4, Irx4, and Nkx2.5.

9. The method of any one of claims 1 to 7, wherein the cells of the cell population that co-express CD49c and CD90 further express at least one trophic factor selected from the group consisting of:
a) Brain-Derived Neurotrophic Factor (BDNF);
b) Cystatin-C;
c) Interleukin-6 (IL-6);
d) Interleukin-7 (IL-7);
e) Interleukin-11 (IL-11);
f) Nerve Growth Factor (NGF);
g) Neurotrophin-3 (NT-3);
h) Macrophage Chemoattractant Protein-1 (MCP-1);
i) Matrix Metalloproteinase-9 (MMP-9);
j) Stem Cell Factor (SCF); and,
k) Vascular Endothelial Growth Factor (VEGF).

10. The method of any one of claims 1 to 7, wherein the cells of the cell population that co-express CD49c and CD90 further express p21 or p53, and wherein expression of p53 is a relative expression of up to 3000 transcripts of p53 per 10⁶ transcripts of an 18s rRNA and expression of p21 is a relative expression of up to 20,000 transcripts of p21 per 10⁶ transcripts of an 18s rRNA.

11. The method of any one of claims 1 to 7, wherein the isolated cell population has been cultured *in vitro* through a number of population doublings selected from the group consisting of:
a) at least 20 population doublings;
b) at least 30 population doublings;
c) at least 40 population doublings; and
d) at least 50 population doublings,
wherein the isolated cell population does not substantially express a senescent marker selected from the group consisting of P21 or P53, or wherein the isolated cell population expresses P53 at a relative expression of up to 3000 transcripts of P53 per 10⁶ transcripts of an 18s rRNA, or wherein the isolated cell population expresses P21 at a relative expression of up to 20,000 transcripts of p21 per 10⁶ transcripts of an 18s rRNA.

12. An isolated cell population derived from human bone marrow, wherein between 100% to 90% of the cells of the cell population co-express CD49c and CD90, and wherein the cell population has a doubling time of less than 30 hours, that can be obtained by the method of any one of claims 1 to 11.

13. The method of claim 1, wherein prior to steps a) and b) in claim 1, the method further comprises the steps of:
a) lysing the red blood cell component of a human bone marrow aspirate;
b) seeding the non-lysed bone marrow cells in a tissue culturing device;
c) allowing the non-lysed bone marrow cells to adhere to a surface of the tissue culturing device;
and wherein steps a) and b) of claim 1 correspond to steps d) and e) comprising:
d) culturing the adherent cells under a 5% oxygen condition; and
e) passaging the adherent cells at a seeding density of 30 cells/cm².

14. An isolated cell population derived from human bone marrow, wherein between 100% to 90% of the cells of the cell population co-express CD49c and CD90, and wherein the cell population has a doubling time of less than 30 hours, that can be obtained by the method of claim 13.

15. The method of claim 1, wherein prior to steps a) and b) in claim 1, the method further comprises the steps of:
a) selecting a fractionated source of the cell population of a human bone marrow aspirate by passage through a density gradient;
b) seeding the fractionated cells in a tissue culturing device;
c) allowing the fractionated cells to adhere to a surface of the tissue culturing device;
and wherein steps a) and b) of claim 1 correspond to steps d) and e) comprising:
d) culturing the adherent cells under a 5% oxygen condition; and
e) passaging the adherent cells at a seeding density of 30 cells/cm².

16. An isolated cell population derived from human bone marrow, wherein between 100% to 90% of the cells of the cell population co-express CD49c and CD90, and wherein the cell population has a doubling time of less than 30 hours, that can be obtained by the method of claim 15.

## Patentansprüche

1. Verfahren zur Herstellung einer isolierten, von menschlichem Knochenmark abgeleiteten Zellpopulation, worin zwischen 100 % und 90 % der Zellen der Zellpopulation CD49c und CD90 ko-exprimieren, und worin die Zellpopulation eine Verdoppelungszeit von weniger als 30 Stunden aufweist, welches die Schritt umfasst:
a) Züchten einer Quelle der Zellpopulation unter Bedingungen mit geringem Sauerstoffgehalt von weniger als 15 % Sauerstoff, um eine adhärente Zellpopulation zu erzeugen; und
b) Züchten der adhärenten Zellpopulation zu einer Aussäh-Dichte von weniger als 2500 Zellen/cm².

2. Verfahren nach Anspruch 1, wobei die Quelle der Zellpopulation in Anspruch 1, Teil a) zu einer Aussäh-Dichte von weniger als 75000 Zellen/cm² gezüchtet wird und wobei die adhärente Zellpopulation in Anspruch 1, Teil b) zu einer Aussäh-Dichte von weniger als 100 Zellen/cm² gezüchtet wird.

3. Verfahren nach Anspruch 1 oder 2, wobei die adhärente Zellpopulation in Anspruch 1, Teil b) unter einer Konzentration an gelöstem Sauerstoff von weniger als 20 % zu einer Aussäh-Dichte gezüchtet wird, ausgewählt aus der Gruppe bestehend aus:
a) weniger als 1000 Zellen/cm²;
b) weniger als 100 Zellen/cm²;
c) weniger als 50 Zellen/cm²; und
d) weniger als 30 Zellen/cm².

4. Verfahren nach einem der Ansprüche 1 bis 3, wobei die Bedingung mit geringem Sauerstoff ausgewählt ist aus der Gruppe bestehend aus:
a) zwischen 1 bis 10 % Sauerstoff,
b) zwischen 2 bis 7 % Sauerstoff,
c) weniger als 10 % Sauerstoff,
d) weniger als 5 % Sauerstoff, und
e) 5 % Sauerstoff.

5. Verfahren nach einem der Ansprüche 1 und 2 bis 4, welches weiter Lysieren der Quelle der Zellpopulation vor dem Züchten der Quelle der Zellpopulation umfasst.

6. Verfahren nach einem der Ansprüche 1 bis 4, welches weiter Auswählen einer fraktionierten Quelle der Zellpopulation mittels Leiten durch einen Dichtegradienten vor dem Züchten der Quelle der Zellpopulation umfasst.

7. Verfahren nach einem der Ansprüche 1 bis 6, wobei die Zellen der Zellpopulation, die CD49c und CD90 ko-exprimieren, CD34 und/oder CD45 nicht exprimieren.

8. Verfahren nach einem der Ansprüche 1 bis 7, wobei die Zellen der Zellpopulation, die CD49c und CD90 ko-exprimieren, weiter mindestens einen Herz-verwandten Transkriptionsfaktor exprimieren, ausgewählt aus der Gruppe bestehend aus GATA-4, IRX4 und Nkx2.5.

9. Verfahren nach einem der Ansprüche 1 bis 7, wobei die Zellen der Zellpopulation, die CD49c und CD90 ko-exprimieren, weiter mindestens einen trophen Faktor exprimieren ausgewählt aus der Gruppe bestehend aus:
a) Hirn-abgeleitetem neurotrophen Faktor (BDNF);
b) Cystatin-C;
c) Interleukin-6 (IL-6);
d) Interleukin-7 (IL-7);
e) Interleukin-11 (IL-11);
f) Nervenwachstumsfaktor (NGF);
g) Neurotrophin-3 (NT-3);
h) Makrophagen Chemoanlockungs-Protein-1 (MCP-1);
i) Matrix-Metalloproteinase-9 (MM-9);
j) Stammzellfaktor (SCF); und
k) Vaskular-Endothel-Wachstumsfaktor (VEGF).

10. Verfahren nach einem der Ansprüche 1 bis 7, wobei die Zellen der Zellpopulation, die CD49c und CD90 ko-exprimieren weiter p21 oder p53 exprimieren, und wobei die Expression von p53 eine relative Expression von bis zu 3000 Transkripten von p53 pro 10⁶ Transkripten einer 18s rRNA ist, und wobei die Expression von p21 eine relative Expression von bis zu 20000 Transkripten von p21 pro 10⁶ Transkripten einer 18s rRNA ist.

11. Verfahren nach einem der Ansprüche 1 bis 7, wobei die isolierte Zellpopulation in vitro durch eine Anzahl von Populationsverdoppelungen gezüchtet wurde, ausgewählt aus der Gruppe bestehend aus:
a) mindestens 20 Populationsverdoppelungen;
b) mindestens 30 Populationsverdoppelungen;
c) mindestens 40 Populationsverdoppelungen;
d) mindestens 50 Populationsverdoppelungen;
worin die isolierte Zellpopulation im Wesentlichen keinen Alterungsmarker ausgewählt aus der Gruppe bestehend aus P21 oder P53 exprimiert, oder wobei die isolierte Zellpopulation P53 in einer relativen Expression von bis zu 3000 Transkripten von P53 pro 10⁶ Transkripten einer 18s rRNA exprimiert, oder wobei die isolierte Zellpopulation P21 in einer relativen Expression von bis zu 20000 Transkripten von p21 pro 10⁶ Transkripten einer 18s rRNA exprimiert.

12. Isolierte, von menschlichem Knochenmark abgeleitete Zellpopulation, worin zwischen 100 % und 90 % der Zellen der Zellpopulation CD49c und CD90 ko-exprimieren, und worin die Zellpopulation eine Verdoppelungszeit von weniger als 30 Stunden aufweist, erhältlich nach einem Verfahren nach einem der Ansprüche 1 bis 11.

13. Verfahren nach Anspruch 1, wobei vor den Schritten a) und b) in Anspruch 1 das Verfahren weiter die Schritte umfasst:
a) Lysieren der roten Blutzellen-Komponenten eines menschlichen Knochenmark-Aspirats;
b) Aussähen nicht-lysierter Knochenmarkszellen in einer Gewebekultur-Einrichtung;
c) Ermöglichen, dass die nicht-lysierten Knochenmarkszellen an einer Oberfläche der Gewebekultur-Einrichtung adhärieren;
und wobei die Schritte a) und b) von Anspruch 1 den Schritten d) und e) entsprechen und umfassen:
d) Züchten der adhärenten Zellen bei 5 % Sauerstoff; und
e) Überführen der adhärenten Zellen in einer Aussäh-Dichte von 30 Zellen/cm².

14. Isolierte, von menschlichem Knochenmark abgeleitete Zellpopulation, worin zwischen 100 % und 90 % der Zellen der Zellpopulation CD49c und CD90 ko-exprimieren, und worin die Zellpopulation eine Verdoppelungszeit von weniger als 30 Stunden aufweist, erhältlich nach dem Verfahren gemäß Anspruch 13.

15. Verfahren nach Anspruch 1, wobei vor Schritt a) und b) in Anspruch 1 das Verfahren weiter die Schritte umfasst:
a) Auswählen einer fraktionierten Quelle der Zellpopulation eines menschlichen Knochenmark-Aspirats mittels Leiten durch einen Dichtegradienten;
b) Aussähen der frakionierten Zellen in einer Gewebekultur-Einrichtung;
c) Ermöglichen, dass die nicht-lysierten Knochenmarkszellen an einer Oberfläche der Gewebekultur-Einrichtung adhärieren;
und wobei die Schritte a) und b) von Anspruch 1 den Schritten d) und e) entsprechen und umfassen:
d) Züchten der adhärenten Zellen bei 5 % Sauerstoff; und
e) Überführen der adhärenten Zellen in einer Aussäh-Dichte von 30 Zellen/cm².

16. Isolierte, von menschlichem Knochenmark abgeleitete Zellpopulation, worin zwischen 100 % und 90 % der Zellen der Zellpopulation CD49c und CD90 ko-exprimieren, und worin die Zellpopulation eine Verdoppelungszeit von weniger als 30 Stunden aufweist, erhältlich nach dem Verfahren gemäß Anspruch 15.

## Revendications

1. Procédé de préparation d'une population de cellules isolées dérivée de moelle osseuse humaine, dans lequel entre 100 % et 90 % des cellules de la population de cellules coexpriment CD49c et CD90, et dans lequel la population de cellules a un temps de doublement de moins de 30 heures, comprenant les étapes de :
a) mise en culture d'une source de la population de cellules dans une condition de faible teneur en oxygène de moins de 15 % d'oxygène pour produire une population de cellules adhérentes ; et,
b) mise en culture de la population de cellules adhérentes à une densité d'ensemencement de moins de 2 500 cellules/cm².

2. Procédé selon la revendication 1, dans lequel la source de la population de cellules dans la revendication 1, partie a) est mise en culture à une densité d'ensemencement de moins de 75 000 cellules/cm² et la population de cellules adhérentes dans la revendication 1, partie b) est mise en culture à une densité d'ensemencement de moins de 100 cellules/cm².

3. Procédé selon l'une quelconque des revendications 1 à 2, dans lequel la population de cellules adhérentes dans la revendication 1, partie b) est mise en culture sous une concentration en oxygène dissous de moins de 20 % à une densité d'ensemencement choisie dans le groupe consistant en :
a) moins de 1 000 cellules/cm² ;
b) moins de 100 cellules/cm² ;
c) moins de 50 cellules/cm² ; et,
d) moins de 30 cellules/cm².

4. Procédé selon l'une quelconque des revendications 1 à 3, dans lequel la condition de faible teneur en oxygène est choisie dans le groupe consistant en :
a) entre 1 et 10 % d'oxygène ;
b) entre 2 et 7 % d'oxygène ;
c) moins de 10 % d'oxygène ;
d) moins de 5 % d'oxygène ; et,
e) 5 % d'oxygène.

5. Procédé selon l'une quelconque des revendications 1, et 2 à 4, incluant en outre la lyse de la source de la population de cellules avant mise en culture de la source de la population de cellules.

6. Procédé selon l'une quelconque des revendications 1 à 4, incluant en outre la sélection d'une source fractionnée de la population de cellules par passage à travers un gradient de densité avant mise en culture de la source de la population de cellules.

7. Procédé selon l'une quelconque des revendications 1 à 6, dans lequel les cellules de la population de cellules qui coexpriment CD49c et CD90 n'expriment pas CD34 et/ou CD45.

8. Procédé selon l'une quelconque des revendications 1 à 7, dans lequel les cellules de la population de cellules qui coexpriment CD49c et CD90 expriment en outre au moins un facteur de transcription lié au coeur choisi dans le groupe consistant en GATA-4, Irx4 et Nkx2.5.

9. Procédé selon l'une quelconque des revendications 1 à 7, dans lequel les cellules de la population de cellules qui coexpriment CD49c et CD90 expriment en outre au moins un facteur trophique choisi dans le groupe consistant en :
a) facteur neurotrophique dérivé du cerveau (BDNF) ;
b) cystatine C ;
c) interleukine-6 (IL-6) ;
d) interleukine-7 (IL-7) ;
e) interleukine-11 (IL-11) ;
f) facteur de croissance du tissu nerveux (NGF) ;
g) neurotrophine 3 (NT-3) ;
h) protéine de chimiotactique des macrophages de type 1 (MCP-1) ;
i) métalloprotéinase matricielle 9 (MMP-9) ;
j) facteur de cellules souches (SCF) ; et
k) facteur de croissance endothéliale vasculaire (VEGF).

10. Procédé selon l'une quelconque des revendications 1 à 7, dans lequel les cellules de la population de cellules qui coexpriment CD49c et CD90 expriment en outre p21 ou p53, et dans lequel l'expression de p53 est une expression relative allant jusqu'à 3 000 transcrits de p53 pour 10⁶ transcrits d'un ARNr 18s et l'expression de p21 est une expression relative allant jusqu'à 20 000 transcrits de p21 pour 10⁶ transcrits d'un ARNr 18s.

11. Procédé selon l'une quelconque des revendications 1 à 7, dans lequel la population de cellules isolées a été mise en culture *in vitro* par un certain nombre de doublements de population choisis dans le groupe consistant en :
a) au moins 20 doublements de population ;
b) au moins 30 doublements de population ;
c) au moins 40 doublements de population ; et
d) au moins 50 doublements de population,
dans lequel la population de cellules isolées n'exprime sensiblement pas de marqueur de sénescence choisi dans le groupe consistant en P21 ou P53, ou dans lequel la population de cellules isolées exprime P53 à une expression relative allant jusqu'à 3 000 transcrits de P53 pour 10⁶ transcrits d'un ARNr 18s, ou dans lequel la population de cellules isolées exprime P21 a une expression relative allant jusqu'à 20 000 transcrits de p21 pour 10⁶ transcrits d'un ARNr 18s.

12. Population de cellules isolées dérivée de moelle osseuse humaine, dans laquelle entre 100 % et 90 % des cellules de la population de cellules coexpriment CD49c et CD90, et dans laquelle la population de cellules a un temps de doublement de moins de 30 heures, qui peut être obtenue par le procédé selon l'une quelconque des revendications 1 à 11.

13. Procédé selon la revendication 1, dans lequel avant les étapes a) et b) dans la revendication 1, le procédé comprend en outre les étapes de :
a) lyse du composant de globule rouge d'un aspirat de moelle osseuse humaine ;
b) ensemencement des cellules de moelle osseuse non lysées dans un dispositif de mise en culture de tissu ;
c) fait de laisser les cellules de moelle osseuse non lysées adhérer à une surface du dispositif de mise en culture de tissu ;
et dans lequel les étapes a) et b) de la revendication 1 correspondent aux étapes d) et e) comprenant :
d) la mise en culture des cellules adhérentes dans une condition d'oxygène à 5 % ; et
e) le passage des cellules adhérentes à une densité d'ensemencement de 30 cellules/cm².

14. Population de cellules isolées dérivée de moelle osseuse humaine, dans laquelle entre 100 % et 90 % des cellules de la population de cellules coexpriment CD49c et CD90, et dans laquelle la population de cellules a un temps de doublement de moins de 30 heures, qui peut être obtenue par le procédé selon la revendication 13.

15. Procédé selon la revendication 1, dans lequel avant les étapes a) et b) dans la revendication 1, le procédé comprend en outre les étapes de :
a) sélection d'une source fractionnée de la population de cellules d'un aspirat de moelle osseuse humaine par passage à travers un gradient de densité ;
b) ensemencement des cellules fractionnées dans un dispositif de mise en culture de tissu ;
c) fait de laisser les cellules fractionnées adhérer à une surface du dispositif de mise en culture de tissu ;
et dans lequel les étapes a) et b) de la revendication 1 correspondent aux étapes d) et e) comprenant :
d) la mise en culture des cellules adhérentes dans une condition d'oxygène à 5 % ; et
e) le passage des cellules adhérentes à une densité d'ensemencement de 30 cellules/cm².

16. Population de cellules isolées dérivée de moelle osseuse humaine, dans laquelle entre 100 % et 90 % des cellules de la population de cellules coexpriment CD49c et CD90, et dans laquelle la population de cellules a un temps de doublement de moins de 30 heures, qui peut être obtenue par le procédé selon la revendication 15.


### SEQUENCE LISTING

<110> Neuronyx, Inc.
   Ho, Tony W.
   Kopen, Gene C.
   Righter, William F.
   Rutkowski, J. Lynn
   Wagner, Joseph
   Herring, W. Joseph
   Ragalia, Vanessa
<120> CELL POPULATIONS WHICH CO-EXPRESS CD49c AND CD90
<130> 2831.2003003
<150> 09/960,244 <151> 2001-09-21
<160> 16
<170> FastSEQ for Windows Version 4.0
<210> 1
   <211> 19
   <212> DNA
   <213> Artificial Sequence
<220>
   <223> Oligonucleotide primers
<400> 1
   atggggatcg gggattgca 19
<210> 2
   <211> 20
   <212> DNA
   <213> Artificial Sequence
<220>
   <223> Oligonucleotide primers
<400> 2
   ccgatccgag ggcctcacta 20
<210> 3
   <211> 26
   <212> DNA
   <213> Artificial Sequence
<220>
   <223> Oligonucleotide primers
<400> 3
   cactccagtt gtccccacag tagaca 26
<210> 4
   <211> 22
   <212> DNA
   <213> Artificial Sequence
<220>
   <223> Oligonucleotide primers
<400> 4
   tcgctttcca tgtgtgaggt ga 22
<210> 5
   <211> 20
   <212> DNA
   <213> Artificial Sequence
<220>
   <223> Oligonucleotide primers
<400> 5
   ggccggagtg gacgaggcaa 20
<210> 6
   <211> 26
   <212> DNA
   <213> Artificial Sequence
<220>
   <223> Oligonucleotide primers
<400> 6
   catcaagctt ctgtctgtgc cttctg 26
<210> 7
   <211> 21
   <212> DNA
   <213> Artificial Sequence
<220>
   <223> Oligonucleotide primers
<400> 7
   accgaggcac tcagaggagg c 21
<210> 8
   <211> 21
   <212> DNA
   <213> Artificial Sequence
<220>
   <223> Oligonucleotide primers
<400> 8
   gccattagcg catcacagtc g 21
<210> 9
   <211> 25
   <212> DNA
   <213> Artificial Sequence
<220>
   <223> Oligonucleotide primers
<400> 9
   gatgttttgc caactggcca agacc 25
<210> 10
   <211> 25
   <212> DNA
   <213> Artificial Sequence
<220>
   <223> Oligonucleotide primers
<400> 10
   aggaggggcc agaccatcgc tatct 25
<210> 11
   <211> 26
   <212> DNA
   <213> Artificial Sequence
<220>
   <223> Oligonucleotide primers
<400> 11
   acaacgaacg ccgcttcctc aggaac 26
<210> 12
   <211> 23
   <212> DNA
   <213> Artificial Sequence
<220>
   <223> Oligonucleotide primers
<400> 12
   gccggaacac agccaacccc tgg 23
<210> 13
   <211> 25
   <212> DNA
   <213> Artificial Sequence
<220>
   <223> Oligonucleotide primers
<400> 13
   ggcagctaca gcatgatgca ggacc 25
<210> 14
   <211> 24
   <212> DNA
   <213> Artificial Sequence
<220>
   <223> Oligonucleotide primers
<400> 14
   ctggtcatgg agttgtactg cagg 24
<210> 15
   <211> 20
   <212> DNA
   <213> Artificial Sequence
<220>
   <223> Oligonucleotide primers
<400> 15
   caagatggtg actcgaacga 20
<210> 16
   <211> 20
   <212> DNA
   <213> Artificial Sequence
<220>
   <223> Oligonucleotide primers
<400> 16
   ggttttgtca aacatcagca 20